# DEMANDE DE BREVET EUROPEEN

(11) **EP 1 087 019 A1**
(43) Date de publication de la demande: **28.03.2001**
(21) Numéro de dépôt: 00402401.4
(22) Date de dépôt: 31.08.2000
(51) Int. Cl.: C12Q 1/04

(54) **Méthode de détection des mycobactéries**

(30) Priorité: 02.09.1999 FR 9911017
(71) Demandeur: Stago International, 92600 Asnières (FR)
(72) Inventeur: Contant, Geneviève, 92400 Courbevoie (FR); Maussion, Anne, 93120 La Courneuve (FR); Simon, Bénédicte, 92400 Courbevoie (FR)
(74) Mandataire: Warcoin, Jacques

(57) **Abrégé**

La présente invention concerne un procédé de dépistage et de différenciation de souches de mycobactéries. Plus précisément, l'invention permet le dépistage et la différenciation des mycobactéries atypiques non pathogènes des mycobactéries responsables de pathologies respiratoires, et, au sein de ces dernières, la détection spécifique du complexe *M*. *tuberculosis.*

## Description

L'invention concerne un procédé de dépistage et de différenciation de souches de mycobactéries. Plus précisément, l'invention permet le dépistage et la différenciation des mycobactéries atypiques non pathogènes des mycobactéries responsables de pathologies respiratoires, et, au sein de ces dernières, la détection spécifique du complexe *M. tuberculosis.*

La tuberculose représente, parmi les maladies infectieuses dues à un seul germe, la principale cause de morbidité et de mortalité (3, 20). Environ un tiers de la population mondiale est infectée par *M. tuberculosis.* En 1988, 8 millions de nouveaux cas ont été diagnostiqués, entraînant trois millions de décès (22). En l'an 2000, plus de 10 millions de cas annuels sont attendus (3). La majorité des cas sont observés dans les pays en voie de développement mais la tuberculose demeure un problème de santé publique dans un grand nombre de pays industrialisés. En France, environ 9000 nouveaux cas sont observés par an (25). La majorité des cas déclarés sont des cas pulmonaires. Les infections à mycobactéries du complexe *M*. *avium-M. intracellulare* (MAC) ont été observées très fréquemment chez les malades atteints de syndrome d'immuno-dépression acquise dans les 15 dernières années (12). Les autres mycobactéries atypiques potentiellement pathogènes, essentiellement *M. xenopi* et *M. kansasii* peuvent provoquer des atteintes pulmonaires (11, 19).

Pour prévenir la dissémination de la tuberculose, le Centre de Contrôle et de Prévention des Maladies (CDC) recommande d'employer les méthodes de diagnostic bactériologique les plus rapides possible afin de rendre les résultats de la culture et de l'identification dans un délai maximal de 21 jours (1, 24) et ceux de sensibilité aux antibiotiques dans un délai maximal de 28 jours après le prélèvement, ceci d'autant plus que des souches multi-résistantes, difficiles à traiter, émergent (2, 9). Pour ce faire, il recommande d'utiliser une combinaison de milieux liquides et solides (16).

Plusieurs méthodes sont utilisées pour la détection des mycobactéries dans les échantillons respiratoires (5, 15, 21, 26) : des méthodes classiques (microscopie et culture) et des méthodes récentes de biologie moléculaire.

La mise en évidence de bacilles acido-alcoolo-résistants ou BAAR au microscope est la méthode la plus rapide mais elle n'est pas sensible (15) et elle n'est pas spécifique d'espèce.

La culture est environ deux fois plus sensible que l'examen microscopique mais elle est longue du fait du temps de doublement des mycobactéries et peut être entravée par des problèmes de contaminations (6). Le délai de détection de la croissance de *M*. *tuberculosis* est de 3 à 8 semaines en milieu solide. Il est réduit en milieu liquide, différents indicateurs précoces de la croissance ayant été proposés dans divers systèmes (Bactec, MGIT, MB Redox, MB Bac T) (4, 5, 6, 17, 23).

Les méthodes de biologie moléculaire sont complémentaires et non substitutives des méthodes conventionnelles. L'amplification génique est théoriquement très sensible (seuil de 1 à 10 UFC/ml) et permet l'obtention des résultats dans la journée mais elle n'a pas toujours fourni dans la pratique des résultats satisfaisants (5).

L'identification classique, qui est basée sur divers tests de croissance et tests biochimiques, est souvent longue et laborieuse (8, 27). Elle peut être remplacée par l'hybridation directe avec une sonde spécifique d'espèce pour quelques espèces : *M*. *tuberculosis, M. avium, M. intracellulare, M. kansasii* et *M. gordonae* (10, 13). Le résultat est alors obtenu en 2 heures.

Le diagnostic différentiel rapide de *M. tuberculosis* des autres espèces est nécessaire compte-tenu du traitement différent des patients en fonction de l'espèce isolée, et du risque de dissémination de *M. tuberculosis.* Des inhibiteurs sélectifs de cette espèce ont été décrits (12, 15) pour la différencier des mycobactéries atypiques.

L'invention propose un procédé conduisant à :
1. Un test de dépistage des mycobactéries plus simple que les méthodes préexistantes,
2. Un test de différenciation des espèces permettant de limiter le nombre de tests d'identification qui sont normalement réalisés pour confirmer ou préciser les résultats obtenus avec les systèmes de dépistage existants et permettant d'obtenir un résultat plus rapidement que les méthodes existantes.

Outre sa spécificité et sa sélectivité, le procédé selon la présente invention est rapide et de mise en oeuvre simple pour le manipulateur, car il est réalisé directement dans les tubes où sont ensemencés les échantillons.

Par ailleurs, le procédé de l'invention ne met en oeuvre aucune étape de centrifugation, ce qui permet de limiter les risques de projection accidentelle.

Le procédé selon l'invention consiste à utiliser le système gélifié de détection de microorganismes tel que décrit dans W096/29427, un tel système étant schématiquement constitué d'un tube comprenant au moins une phase dite de révélation, constituée d'un gel comportant un milieu de culture de microorganismes et un réactif induisant une variation de mesure optique détectable en présence de microorganismes. Un tel système gélifié sera ci-après dénommé « tube gel » ou « tube ». Les tubes gel préférentiellement utilisés dans le cadre de l'invention sont des tubes dont le fond a une partie capillaire qui permet de mieux concentrer localement les bactéries donc de mieux visualiser un changement de couleur situé à ce niveau. La concentration des mycobactéries s'effectue dans le capillaire par sédimentation, sans qu'il soit nécessaire de réaliser une centrifugation.

L'indicateur utilisé dans le cadre de l'invention est un indicateur redox, la résazurine, qui est de couleur bleue non fluorescente dans sa forme oxydée et qui, après une première étape de réduction, se transforme en résorufine, de couleur rose et fluorescente, qui elle-même se transforme en un composé incolore non fluorescent, la dihydroresorufine, après une seconde étape de réduction.

La résazurine est un composé classiquement utilisé pour visualiser la croissance de bactéries en culture. Son taux de réduction, qui dépend de l'activité métabolique des bactéries, est directement proportionnel à la concentration bactérienne et est donc une indication de leur taux de croissance (28).

Dans le cadre de l'invention, on a montré de façon surprenante que la résazurine, dans la gamme de concentrations normalement utilisée pour révéler les bactéries dans les tubes gels, avait un effet inhibiteur sur certaines souches de mycobactéries. En particulier, on a montré que le complexe *M. tuberculosis* était spécifiquement inhibé par des concentrations en résazurine qui n'affectaient pas la croissance des souches de mycobactéries atypiques ni celle des autres espèces pathogènes (CMI en résazurine plus faible pour *M. tuberculosis* par rapport aux autres mycobactéries).

Par ailleurs, on a également observé que la croissance de certaines souches pathogènes était ralentie par la résazurine et que celle-ci potentialisait l'effet d'un inhibiteur spécifique sur toutes les mycobactéries pathogènes, sans pertuber la croissance des espèces non pathogènes.

Partant de ces observations, la méthode selon la présente invention permet de différencier les mycobactéries impliquées en pathologie respiratoire des autres espèces non pathogènes, et/ou de différencier le complexe *M. tuberculosis* des autres espèces de mycobactéries atypiques (pathogènes respiratoires et non pathogènes) et/ou de différencier le complexe *M. tuberculosis,* les mycobactéries atypiques impliquées en pathologie respiratoire et les autres espèces non pathogènes.

On entend par :
- « complexe *M. tuberculosis* » : *M. tuberculosis, M. bovis, M. africanum, M. BCG.*
- « Mycobactéries atypiques » : les mycobactéries atypiques "pathogènes respiratoires" et les mycobactéries atypiques non pathogènes.
- « échantillon biologique » : les prélèvements d'origine pulmonaire, les liquides biologiques prélevés stérilement (urines, liquides d'épanchement, etc.), les selles décontaminées, biopsies et pièces opératoires si nécessaire diluées ou fluidifiées.
- « tube gel » ou « tube » : un tube comprenant au moins une phase dite de révélation, constituée d'un gel comportant un milieu de culture de microorganismes et un réactif induisant une variation de mesure optique détectable en présence de microorganismes. Une description détaillée de ces tubes est accessible à l'homme du métier notamment dans W096/29427.

De ce fait, selon une première variante, l'invention a pour objet une méthode de différenciation du complexe *M. tuberculosis* des autres mycobactéries atypiques dans un échantillon biologique comprenant les étapes consistant à :
a) ensemencer simultanément deux aliquotes d'un échantillon à tester dans
   - un tube gel n° 1, comprenant dans une phase gel stérile :
      (i) un milieu de culture pour mycobactéries additionné d'un mélange d'antibiotiques pour inhiber les bactéries contaminantes,
      (ii) de la résazurine à une concentration C1 inférieure à la CMI pour le complexe *M. tuberculosis* et suffisante pour révéler la présence de microorganismes dans l'échantillon,
   - un tube gel n° 2, comprenant dans une phase gel stérile :
      (i) un milieu de culture pour mycobactéries additionné d'un mélange d'antibiotiques pour inhiber les bactéries contaminantes.
      (ii) soit de la résazurine à une concentration C2 supérieure ou égale à la CMI pour le complexe *M. tuberculosis,* et inférieure à la CMI pour les autres espèces de mycobactéries, soit de la résazurine à une concentration C'2 inférieure ou égale à la CMI pour le complexe *M*. *tuberculosis* à une concentration suffisante pour révéler la présence de microorganismes dans l'échantillon, et un inhibiteur spécifique du complexe *M*. *tuberculosis* à une concentration suffisante pour inhiber sa croissance,
b) agiter immédiatement les tubes 1 et 2 après l'étape d'ensemencement pour mélanger l'échantillon et le gel,
c) incuber les tubes n° 1 et 2 à 37° C et procéder à la lecture des tubes.

Dans le cadre de la présente invention, la CMI, ou Concentration Minimale Inhibitrice d'un composé, est déterminée comme la plus faible concentration de ce composé capable d'inhiber la croissance bactérienne, dans les conditions du tube gel. La CMI d'un composé en tubes gel peut être dérivée de la CMI trouvée pour ce composé par la méthode en milieu liquide, en utilisant comme milieu celui contenu dans les tubes gel. La méthode dite des proportions sur gélose est considérée comme méthode de référence (voir exemples ci-après).

La lecture des tubes est généralement réalisée à partir de deux jours d'incubation, le nombre de jours d'incubation étant fonction de la concentration de mycobactéries initialement présentes dans l'échantillon.

La durée maximale d'incubation est généralement de six à huit semaines. La lecture est réalisée en suivant l'apparition d'une couleur rose au niveau du gel de la partie capillaire.
Préférentiellement, la lecture est réalisée tous les jours pendant deux semaines, puis toutes les semaines.
Si le gel est resté violet quelque soit le tube, l'incubation doit être prolongée

Dans le cas où l'échantillon contient des mycobactéries, celles-ci seront détectées dans le tube 1 (virage du bleu au rose) quelque soit l'espèce présente. Si l'espèce contenue dans l'échantillon appartient au complexe *M*. *tuberculosis,* sa croissance sera inhibée par la plus grande concentration de résazurine présente dans le tube 2. Dans ce cas, le tube 2 restera donc négatif (couleur bleue). Si l'échantillon contient une autre espèce de mycobactérie atypique, celle-ci pourra également pousser dans le tube 2 qui donnera alors un résultat positif (couleur rose), comme le tube 1.

Préférentiellement, la méthode de l'invention comprend les étapes consistant à :
a) ensemencer simultanément deux aliquotes d'un échantillon à tester dans :
   - un tube gel n° 1, comprenant dans une phase gel stérile :
      (i) un milieu de culture pour mycobactéries additionné d'un mélange d'antibiotiques pour inhiber les bactéries contaminantes,
      (ii) de la résazurine à une concentration C1 inférieure à la CMI pour le complexe *M. tuberculosis* et suffisante pour révéler la présence de microorganismes dans l'échantillon.
   - un tube gel n° 2, comprenant dans une phase gel stérile :
      (i) un milieu de culture pour mycobactéries additionné d'un mélange d'antibiotiques pour inhiber les bactéries contaminantes.
      (ii) de la résazurine à une concentration C'2 inférieure ou égale à la CMI pour le complexe *M. tuberculosis* et à une concentration suffisante pour révéler la présence de microorganismes dans l'échantillon, et un inhibiteur spécifique du complexe *M. tuberculosis* à une concentration suffisante pour inhiber sa croissance,
b) agiter immédiatement les tubes 1 et 2 après l'étape d'ensemencement pour mélanger l'échantillon et le gel,
c) incuber les tubes n° 1 et 2 à 37° C et procéder à la lecture.

Ce mode de réalisation s'avère avantageux au niveau de la phase de lecture, car il permet d'éviter des décalages au niveau du temps d'apparition d'une couleur rose entre les tubes 1 et 2 ; ces décalages étant dus à un ralentissement de la croissance de certaines souches en fonction de la concentration de résazurine.

Un composé décrit dans la littérature comme un inhibiteur spécifique de *M. tuberculosis* est par exemple l'hydroxylamine (27, 29) ou un dérivé de l'acide benzoïque tel que le salicylate de sodium (27, 30).

Dans le cadre de la présente invention, on utilise préférentiellement le salicylate de sodium.

Selon une autre variante, l'invention a pour objet une méthode de différenciation des mycobactéries atypiques impliquées en pathologie respiratoire des autres espèces non pathogènes dans un échantillon biologique, comprenant les étapes consistant à :
a) ensemencer simultanément deux aliquotes d'un échantillon à tester dans :
   - un tube gel n° 1, comprenant dans une phase gel stérile :
      (i) un milieu de culture pour mycobactéries additionné d'un mélange d'antibiotiques pour inhiber les bactéries contaminantes,
      (ii) de la résazurine à une concentration C1 inférieure à la CMI pour le complexe *M*. *tuberculosis* et suffisante pour révéler la présence de microorganismes dans l'échantillon.
   - un tube gel n° 3, comprenant dans une phase gel stérile :
      (i) un milieu de culture pour mycobactéries additionné d'un mélange d'antibiotiques pour inhiber les bactéries contaminantes.
      (ii) de la résazurine à l'intérieur du gel à une concentration C3 supérieure à C1 et un inhibiteur spécifique des mycobactéries à une concentration C4, C3 et C4 étant choisies de façon à inhiber les mycobactéries atypiques pathogènes éventuellement présentes dans l'échantillon, sans affecter la croissance des mycobactéries atypiques non pathogènes, et C3 étant une concentration habituellement utilisée pour révéler la présence de microorganismes dans un échantillon,
b) agiter immédiatement les tubes 1 et 3 après l'étape a) d'ensemencement pour mélanger l'échantillon et le gel,
c) incuber les tubes n° 1 et 3 à 37° C et procéder à la lecture des tubes à partir de 2 jours d'incubation, le nombre de jours d'incubation étant fonction de la concentration de mycobactéries initialement présentes dans l'échantillon,

La lecture est réalisée comme indiqué dans la variante précédente.
Dans le cas où l'échantillon contient des espèces de mycobactéries pathogènes, seul le tube 1 sera positif, la croissance des souches étant inhibée dans le tube 3 du fait de la présence de la concentration de résazurine élevée d'une part, et de la présence de l'inhibiteur d'autre part. Si l'échantillon contient des mycobactéries atypiques non pathogènes, le résultat sera positif dans les tubes 1 et 3 (virage du bleu au rose).

L'inhibiteur spécifique des mycobactéries pathogènes préférentiellement utilisé est un dérivé NO2, en particulier le nitrite de sodium, dont les propriétés ont été décrites dans la littérature (27, 31).

Il est évidemment possible dans le cadre de la présente invention de combiner les deux variantes qui précèdent en utilisant dans une même méthode les trois tubes gel 1, 2 et 3.

Dans ce cas, l'invention permet à la fois de détecter la présence de mycobactéries pathogènes, en vérifiant si celles-ci appartiennent ou non au complexe *M. tuberculosis* et de détecter la présence des espèces non pathogènes.

Selon une autre variante avantageuse, l'invention a donc pour objet une méthode de détection et de différenciation des mycobactéries du complexe *M*. *tuberculosis* des mycobactéries pathogènes et des autres espèces atypiques non pathogènes, ladite méthode consistant à :
a) ensemencer simultanément trois aliquotes d'un même échantillon dans des tubes gel 1, 2 et 3 tels que décrits ci-dessus,
b) agiter les tubes immédiatement après ensemencement et,
c) incuber les tubes à 37° C et procéder à la lecture à partir de 2 jours d'incubation.

Si l'échantillon ne contient que des mycobactéries non pathogènes, les trois tubes seront positifs.
Si l'échantillon contient des mycobactéries pathogènes autres que le complexe *M*. *tuberculosis,* les tubes 1 et 2 seront positifs, le tube 3 sera négatif car la croissance de ces mycobactéries aura été inhibée par l'inhibiteur et/ou la concentration élevée de résazurine.

Si l'échantillon contient des mycobactéries du complexe *M. tuberculosis,* seul le tube 1 sera positif. Les tubes 2 et 3 seront négatifs du fait de la présence respectivement d'une concentration de résazurine supérieure à la CMI ou d'un inhibiteur spécifique *de M. tuberculosis* dans le tube n° 2 et d'une concentration de résazurine supérieure à la CMI et d'un inhibiteur spécifique des souches pathogènes dans le tube n° 3.

Il est également possible de n'utiliser que les tubes 2 et 3, si l'on sait au préalable que l'échantillon contient des mycobactéries pathogènes mais dont on ignore l'espèce.

On dispose alors d'une méthode de différenciation des mycobactéries du *complexe M*. *tuberculosis* des autres espèces pathogènes en procédant avec les tubes 2 et 3 tel que cela vient d'être décrit.

Enfin, il est évidemment possible de n'utiliser que le tube 1 pour un simple dépistage de toutes les mycobactéries (pathogènes ou non pathogènes), dont on ne veut pas connaître l'identification.

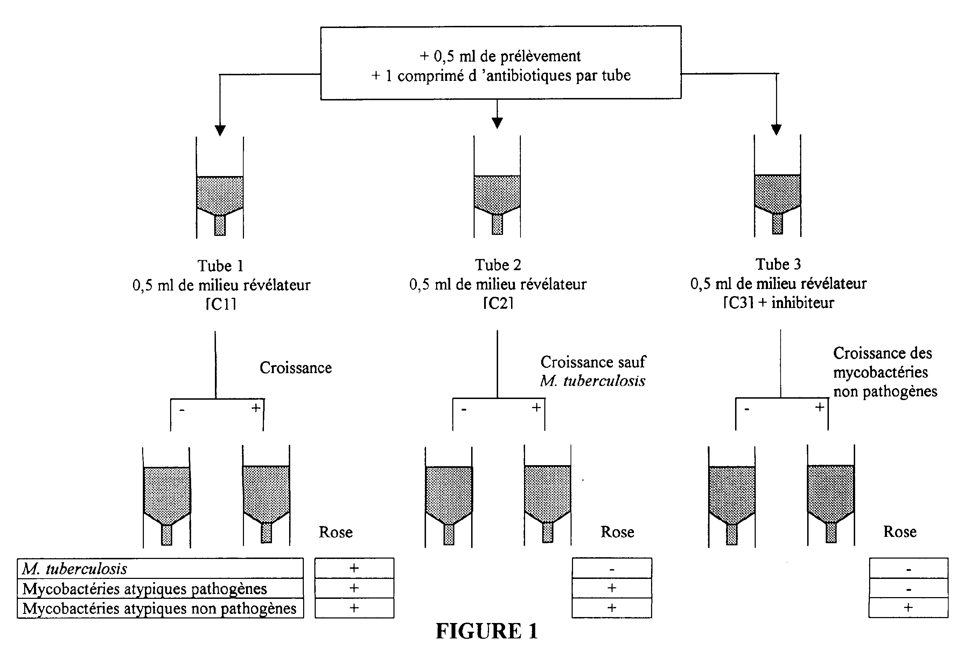

Le principe général de la méthode de l'invention est résumé sur la figure 1 ci-après.

Selon un mode de réalisation avantageux, la méthode de l'invention permet en outre de dépister la présence d'une mycobactérie atypique poussant à 30° C en cas d'infection cutanée.

Dans ce cas, on utilise un tube supplémentaire identique au tube no. 1 mais qui est incubé à 30° C dans l'étape c) définie ci-dessus. Si, au moment de la lecture, celui-ci donne un résultat positif, quelle que soit la positivité des tubes incubés à 37° C, cela fait suspecter la présence d'une bactérie atypique poussant à 30° C.

La concentration C1 finale en résazurine dans la phase gel du tube no. 1 tel que décrit précédemment est avantageusement comprise entre 18 et 25 mg/l. Préférentiellement, cette concentration est de 22 mg/l.

La concentration C2 finale en résazurine dans la phase gel du tube no. 2 est avantageusement comprise entre 30 mg/l et 50 mg/l. Elle est préférentiellement comprise entre 40 et 50 mg/l et plus préférentiellement égale à 45 mg/l.

Dans le cas où on utilise dans le tube gel n° 2 un inhibiteur spécifique du complexe *M*. *tuberculosis,* la concentration C'2 en résazurine est inférieure ou égale à la CMI pour *M*. *tuberculosis,* et est préférentiellement égale à C1. La concentration de l'inhibiteur du complexe *M*. *tuberculosis* dépend de l'inhibiteur choisi et peut être facilement déterminée par l'homme du métier. Lorsque on utilise du salicylate de sodium, sa concentration dans la phase gel est préférentiellement de 62,5 mg/l final pour une concentration en résazurine de 22 mg/l.

La concentration C3 en résazurine dans la phase gel du tube n° 3 est supérieure à la CMI en résazurine pour le complexe *M. tuberculosis* et inférieure à la CMI pour les mycobactéries atypiques non pathogènes. Elle est avantageusement comprise entre 40 et 50 mg/l et préférentiellement égale à C2. La concentration C4 de l'inhibiteur des mycobactéries pathogènes est supérieure ou égale à sa CMI déterminée dans les conditions du tube gel. Elle est avantageusement comprise entre 0,2 et 0,5 g/l lorsque l'inhibiteur utilisé est le NaNO2. Des concentrations préférées du mélange résazurine/NaNO2 sont respectivement de 45 mg/l et 0,5 g/l. Ces concentrations sont résumées dans le tableau I ci-dessous.

**Tableau I**

| Produit | Tube no. | ( ) dans le tube prêt à l'emploi. | ( ) dans le tube après ajout du prélèvement en tampon phosphate ( ) finale. |
|---|---|---|---|
| Résazurine | T1 | C1 : 44 mg/l | C1 : 22 mg/l |
| | T2 | C2 : 90 mg/l | C2 : 45 mg/l |
| | | ou C'2 : 45 mg/l | ou C'2 : 22 mg/l |
| | T3 | C3 : 90 mg/l | C3 : 45 mg/l |
| Salicylate de sodium | T2 | 125 mg/l | 62,5 mg/l |
| Nitrite de sodium | T3 | 1 g/l | 0,5 g/l |

Outre les constituants essentiels cités ci-dessus, les tubes gel utilisés contiennent un milieu liquide communément utilisé pour la culture des mycobactéries, constitué par un bouillon de base Middlebrook 7H9 enrichi par de l'OADC. Un mélange d'acide oléique, d'albumine, de dextrose et de catalase (O.A.D.C.). Il est possible d'ajouter une source de carbone complémentaire à l'albumine comme l'hydrolysat de caséine pour augmenter la croissance des souches de *M*. *tuberculosis* résistants à l'isoniaside. Le glycérol du milieu favorise la croissance des souches du complexe *M. avium - M*. *intracellulare* (MAC) et le pyruvate de sodium est nécessaire à la croissance de *M*. *bovis.* L'acide oléique est nécessaire à la croissance de *M*. *xenopi* et MAC. Par ailleurs, sont également ajoutés des antibiotiques pour inhiber la croissance d'autres bactéries contaminantes. Un mélange d'ATB préféré est constitué par :
1. du mélange PANTA (polymyxine B, amphotéricine B, acide nalidixique, triméthoprime et azlocilline), utilisé notamment dans le système de radiométrie BACTEC et le tube MGIT de Becton Dickinson,
2. avec un colorant comme le vert de malachite, fréquemment utilisé dans les milieux sélectifs des bactéries (voirCRE colorant + antibiotiques),
3. avec un inhibiteur des cocci Gram positif : préférentiellement le vancomycine utilisée à la concentration finale de 0,5 mg/l (C) dans le tube après ajout du prélèvement en tampon phosphate),
4. et un inhibiteur des bacilles Gram négatif : préférentiellement une cephalosporine de 3ème génération comme le céfotoxine utilisé à la concentration finale de 2 mg/l.

Les antibiotiques sont préférentiellement formulés sous forme de comprimés reconstitués dans la phase gel extemporanément au moment de l'ajout du prélèvement. Il est également avantageux d'ajouter du sérum de poulain pour activer la croissance des mycobactéries dans les tubes. Ceci permet de diminuer les délais de réponse lorsque les inoculi sont faibles. Ce sérum (généralement 5 % par tube) est préférentiellement compris dans le comprimé d'antibiotiques, cette formulation permettant de pallier tout problème d'instabilité du sérum en milieu liquide et de contamination.

Le virage de la couleur des échantillons dans les tubes gel est en général facilement détectable visuellement. Toutefois, la lecture peut être également réalisée avantageusement à l'aide d'un dispositif automatique de détection qui permet notamment de résoudre les problèmes d'interprétation des résultats et des risques de faux négatifs lorsque les échantillons contiennent un nombre très faible de bactéries. Un tel procédé peut être définit en ce qu'il consiste à exécuter, au cours d'une période de temps relativement courte, de l'ordre de la milliseconde à la dizaine de millisecondes, une séquence opératoire comprenant au moins les étapes suivantes :
- une première exposition de l'échantillon à une première impulsion lumineuse incidente (IL₁) dont les ondes lumineuses sont comprises dans une première gamme de fréquence, cette impulsion (IL₁) étant émise par une première source lumineuse optoélectronique (SL₁),
- une première mesure de type turbidimétrique, par un détecteur optoélectronique (DO) situé sensiblement dans l'axe de ladite première impulsion incidente (IL₁) de l'intensité lumineuse transmise de ladite première implusion, après son passage au travers dudit échantillon,
- le stockage du résultat de cette première mesure dans une mémoire (MEM),
- l'exposition de l'échantillon à une seconde impulsion lumineuse incidente (IL₂) présentant sensiblement la même incidence que la première, dont les ondes lumineuses sont comprises dans une deuxième gamme de fréquence, cette implusion (IL₂) étant émise par une seconde source lumineuse optoélectronique (SL₂),
- une deuxième mesure de l'intensité transmise de ladite deuxième impulsion après son passage au travers dudit échantillon,
- l'analyse des résultats stockés dans ladite mémoire.

Dans un autre aspect, l'invention se rapporte à un kit pour la mise en oeuvre d'une méthode définie ci-dessus. Le kit selon l'invention peut être caractérisé en ce qu'il comprend un tube gel n°1 supplémentaire pour la détection de mycobactéries poussant à 30°C.
Les exemples ci-après illustrent la présente invention.

### EXEMPLE 1 : Détermination de la CMI du révélateur utilisé (résazurine) pour différentes souches de mycobactéries pathogènes et non pathogènes.

### 1. Objectif

**a)** Déterminer pour chaque souche de mycobactérie, la CMI du révélateur utilisé dans les tubes gel du test de dépistage-différenciation par la méthode des proportions.
   Le révélateur permet de visualiser la croissance des mycobactéries par un changement de coloration du violet au rose.
   Cependant, le révélateur peut inhiber la croissance de *M*. *tuberculosis* ou d'autres souches proportionnellement à sa concentration.
   Il est donc nécessaire de déterminer la plus faible concentration de révélateur, inhibitrice de la croissance des différentes souches de mycobactéries, en utilisant la méthode des proportions considérée comme méthode de référence.
**b)** Déterminer en parallèle la CMI du révélateur en milieu liquide pour chaque souche de mycobactérie. Les CMI doivent être déterminées dans les conditions du tube gel, en utilisant comme milieu celui contenu dans les 3 tubes gel.
**c)** Déterminer la concentration du révélateur à ajouter dans chacun des 3 tubes gel du test de dépistage-différenciation des mycobactéries :
   - dans le tube gel 1, le révélateur doit être présent à une concentration permettant la croissance de toutes les mycobactéries,
   - dans le tube gel 2, le révélateur en absence d'inhibiteur spécifique de *M. tuberculosis* doit être présent à une concentration qui inhibe la croissance de *M*. *tuberculosis* sans inhiber totalement la croissance des autres espèces,
   - dans le tube gel 3, la différenciation des espèces dépend de la concentration de l'inhibiteur spécifique. : la concentration du révélateur peut être la même que celle du tube gel 1, elle est de préférence supérieure à la concentration du tube gel 1.

### 2. Matériels et méthodes

### 2.1. Souches testées (n = 26)

**Tableau 2 :**

| **liste des souches de l'étude** | |
|---|---|
| **Espèce** | **Nombre de souches testées** |
| *M. tuberculosis* | 6 |
| | |

| **Mycobactéries atypiques pathogènes :** | |
|---|---|
| *M*. *avium* | 4 |
| *M*. *intracellulare* | 1 |
| *M. kansasii* | 5 |
| *M*. *xenopi* | 5 |
| | |

| **Mycobactéries atypiques non pathogènes :** | |
|---|---|
| *M*. *abscessus* | 1 |
| *M*. *chelonae* | 1 |
| *M*. *fortuitum* | 1 |
| *M*. *peregrinum* | 1 |
| *M*. *gordonae* | 1 |

### 2.2.A. Détermination de la CMI du révélateur par la méthode des proportions

La CMI du révélateur vis-à-vis des 26 souches testées a été déterminée par la méthode des proportions (méthode de référence) en milieu 7H11 contenant 10 % d'OADC.

(La concentration minimale inhibitrice (CMI) du révélateur vis-à-vis d'une souche est définie comme la plus faible concentration pour laquelle le nombre de colonies sur la gélose est inférieur à 1 % par rapport au nombre de colonies présentes sur la gélose témoin sans révélateur).
**a) Préparation d'une culture en milieu liquide**
A partir d'une culture de mycobactéries sur milieu Lowenstein-Jensen, quelques colonies sont prélevées à l'aide d'une öese stérile.
Les colonies sont mises dans un tube falcon contenant des billes de verre. Après agitation manuelle pendant 1 à 2 minutes, 2 ml de milieu 7H9 contenant 0.05 % de tween 80 sont ajoutés. Après homogénéisation, le milieu contenant les bactéries est transvasé dans un tube en verre ; 3 ml de milieu 7H9 contenant 0.05 % de tween 80 et 0.5 ml d'OADC (concentration finale : 10 %) sont alors ajoutés.
Les tubes sont mis à l'étuve à :
* 37°C pendant 2 ou 3 jours pour :
   - *M. abscessus*
   - *M. fortuitum*
   - *M. peregrinum*
   - *M. smegmatis ;*
pendant 7 ou jours pour
- *M*. *avium*
- *M. intracellulare*
- *M*. *kansasii*
- *M. tuberculosis ;*
pendant 10 jours pour
- *M*. *xenopi*
* 30°C pour :
- *M*. *chelonae* pendant 2 ou 3 jours
- *M*. *gordonae* pendant 5 ou 6 jours

Le but est d'obtenir une culture dont l'opacité est au moins équivalente à celle du tube BCG à 1 mg/ml (Diagnostics pasteur - Réf. : 53211).
**b) Préparation des inoculi**
L'opacité de la culture de mycobactéries est ajustée à celle du tube BCG à 1 mg/ml en utilisant du milieu 7H9.
Deux dilutions de cet inoculum sont alors réalisées en milieu 7H9 : une dilution à 10⁻³ et une dilution à 10⁻⁵.
Le but est d'obtenir deux inoculi de la culture de mycobactéries :
un inoculum à 10⁻³ et un inoculum à 10⁻⁵.
**c) Préparation des géloses contenant le révélateur à différentes concentrations**
Les géloses doivent contenir 10 % d'OADC et 1 % d'une solution de révélateur à une concentration 100 fois supérieure à la concentration finale désirée.
Les différentes concentrations de révélateur testées sont les suivantes :
15 mg/l - 20 mg/l - 25 mg/l - 30 mg/l - 35 mg/l - 36 mg/l - 37 mg/l - 38 mg/l - 39 mg/l - 40 mg/l - 41 mg/l - 42 mg/l - 43 mg/l - 44 mg/l - 45 mg/l - 50 mg/l et 55 mg/l.
Préparation de la gamme de concentration du révélateur :
Une gamme de différentes concentrations du révélateur de 1.5 à 5.5 mg/ml est préparée à partir d'une solution mère à 6 mg/ml en eau distillée stérile.
Cette gamme est donnée dans le tableau III suivant :

**Tableau III**

| **Concentration en révélateur (mg/ml)** | **Volume de la solution mère (µl)** | **Volume d'eau distillée stérile (µl)** |
|---|---|---|
| 1,5 | 250 | 750 |
| 2,0 | 333 | 666 |
| 2,5 | 417 | 583 |
| 3,0 | 500 | 500 |
| 3,5 | 583 | 417 |
| 3,6 | 600 | 400 |
| 3,7 | 617 | 383 |
| 3,8 | 633 | 367 |
| 3,9 | 650 | 350 |
| 4,0 | 667 | 333 |
| 4,1 | 683 | 317 |
| 4,2 | 700 | 300 |
| 4,3 | 717 | 283 |
| 4,4 | 733 | 267 |
| 4,5 | 750 | 250 |
| 5,0 | 833 | 167 |
| 5,5 | 917 | 83 |

Préparation des géloses contenant les différentes concentrations de révélateur :
Pour chaque souche testée, 400 ml de gélose sont préparés ; 8,4 g de gélose 7H11 sont mélangés à 360 ml d'eau distillée et 2 ml de glycérol. Le mélange est alors porté à ébullition de façon à obtenir une solution translucide.
Après stérilisation (autoclave 15 min à 121°C), la solution est ramenée à une température de 50 - 55°C ; 40 ml d'OADC sont alors additionnés et le tout est maintenu en surfusion à une température de 50 - 55°C.
200 µl de chaque concentration de révélateur sont mélangés de façon homogène à 20 ml du milieu gélosé maintenu en surfusion ; on obtient donc des concentrations finales de révélateur de 15 à 55 mg/l. Le mélange est alors réparti en boîte de pétri à 4 compartiments, à raison de 5 ml par compartiment.
Une gélose ne contenant pas de révélateur est également préparée (témoin de croissance).
**d) Inoculation des géloses contenant le révélateur**
Sur chaque gélose ainsi préparée, l'inoculum 10-3 est déposé sur 2 quadrants à raison de 50 µl par quadrant, et l'inoculum 10-5 est déposé sur 2 quadrants à raison de 50 µl par quadrant. Les cultures sont alors étalées à l'aide d'un râteau. Les géloses ainsi inoculées sont laissées sous la hotte pendant environ 10 minutes afin de les laisser sécher.

Les boîtes sont alors mises dans un sac à autoclave et placées à l'étuve à :
* 37°C pendant 3 et 7 jours pour :
   - *M*. *abscessus*
   - *M*. *fortuitum*
   - *M*. *peregrinum*
   - *M*. *smegmatis*
* 37°C pendant 15 et 21 jours pour :
   - *M*. *avium*
   - *M*. *intracellulare*
   - *M*. *kansasii*
* 37°C pendant 21 et 42 jours pour *M. tuberculosis*
   37°C pendant 28 et 42 jours pour *M*. *xenopi*
* 30°C pendant 3 et 7 jours pour *M*. *chelonae*
   30°C pendant 7 et 15 jour pour *M*. *gordonae*

Si la culture est insuffisante au bout du 1er temps d'incubation, garder les boîtes jusqu'au 2ème temps d'incubation.
Les résultats sont obtenus en comptant le nombre de colonies pour chacun des 2 inoculi sur la gélose témoin de croissance (sans révélateur) et le nombre de colonies pour chacun des 2 inoculi sur les géloses contenant les différentes concentrations en révélateur.

### 2.2.B. Détermination de la CMI du révélateur en milieu liquide

La CMI du révélateur vis-à-vis des 26 souches a été déterminée en milieu liquide (composition du milieu identique à celle des tubes gel 1 et 2) dans un flacon en verre de 6 ml contenant 1 ml de milieu et avec la dilution 10-3 du tube équivalent au tube BCG à 1 mg/ml. La croissance bactérienne est visualisée par le changement de couleur du milieu du violet au rose. La CMI du révélateur vis-à-vis d'une souche est définie comme la plus faible concentration de révélateur inhibant la croissance bactérienne.

La culture en milieu liquide, les inoculi, les milieux contenant le révélateur à différentes concentrations et la gamme de concentration du révélateur ont été décrits précédemment à l'exemple I, partie 2A.

Préparation des milieux contenant les différentes concentrations de révélateur :
Chaque flacon contient :
- 1070 µl d'un milieu dont la composition est identique à celle d'un tube gel,
- et 11 µl de la concentration du révélateur 100 fois concentrée.
Un flacon ne contenant pas de révélateur est également préparé.

Inoculation des milieux contenant le révélateur :
A chaque milieu sont ajoutés 50 µl de l'inoculum 10⁻³.

Les flacons sont mis à l'étuve à :
* 37°C pour :
   - *M*. *abscessus*
   - *M*. *fortuitum*
   - *M*. *peregrinum*
   - *M*. *smegmatis*
   - *M*. *avium*
   - *M*. *intracellulare*
   - *M*. *kansasii*
   - *M*. *tuberculosis*
   - *M*. *xenopi*
* 30°C pour :
   - *M*. *chelonae*
   - *M. gordonae*

La couleur du flacon est notée chaque jour et les flacons sont gardés 6 semaines.

### 3. Résultats des deux méthodes décrites aux points 2.2.A. et 2.2.B. ci-dessus

La CMI du révélateur vis-à-vis des 26 souches de mycobactéries testées, déterminée par les deux méthodes est donnée dans le tableau IV ci-dessous.

**Tableau IV :**

| **résultats de CMI du révélateur vis-à-vis des souches de l'étude** | | | |
|---|---|---|---|
| **Espèce** | **Souche** | **CMI (mg/l) Méthode des proportions** | **CMI (mg/l) Milieu liquide** |
| *M*. *tuberculosis* | H37Rv | 20 | 30 |
| | 4 | ≤15 | 20 |
| | 5 | ≤15 | 25 |
| | 7 | 20 | 36 |
| | 8 | ≤15 | 25 |
| | COET | ≤15 | 25 |
| | | | |

| **Mycobactéries atypiques pathogènes :** | | | |
|---|---|---|---|
| *M*. *avium* | CIP | > 55 | > 55 |
| | BRIG | > 55 | > 55 |
| | ABO | > 55 | > 55 |
| | COD | > 55 | > 55 |
| *M. intracellulare* | CIP | > 55 | > 55 |
| *M*. *kansasii* | ATCC | > 55 | > 55 |
| | VIG | > 55 | ND |
| | MAR | > 55 | ND |
| | HUB | > 55 | ND |
| | VAL | 43 | ND* |
| *M*. *xenopi* | BERN | > 55 | ND |
| | ATA | > 55 | ND |
| | CHI | > 55 | ND |
| | INC | > 55 | ND |
| | BLA | > 55 | ND |

| **Mycobactéries atypiques non pathogènes :** | | | |
|---|---|---|---|
| *M. abscessus* | CIP | > 55 | ND |
| *M. chelonae* | LOE | > 55 | ND |
| *M. fortuitum* | CIP | > 55 | ND |
| *M*. *peregrinum* | CIP | > 55 | ND |
| *M. gordonae* | ANDR | > 55 | > 55 |
| ND : Non déterminée * CMI en cours | | | |

### 3.1. Par la méthode des proportions

- Toutes les souches de *M*. *tuberculosis* sont inhibées par le révélateur à une concentration > 20 mg/l (CMI ( 20 mg/l).
- Toutes les souches des autres espèces ne sont pas inhibées par le révélateur jusqu'à une concentration de 43 mg/l (CMI ( 43 mg/l).
Il est possible de différencier *M*. *tuberculosis* des autres espèces en sélectionnant 2 concentrations différentes de révélateur.
Nous avons remarqué que la taille des colonies était inversement proportionnelle à la concentration du révélateur. Il est préférable de choisir la concentration la plus faible possible.

### 3.2. Par la méthode en milieu liquide

- La CMI du révélateur vis-à-vis des souches de *M. tuberculosis* est ( 36 mg/l. Elle est > 55 mg/l pour les autres espèces.
Il est aussi possible de différencier *M*. *tuberculosis* des autres espèces de la même façon.
Les valeurs de CMI sont plus élevées en milieu liquide qu'en milieu gélosé. Une hypothèse pourrait être donnée par la croissance plus rapide des souches de mycobactéries en milieu liquide au dépend de leur inhibition par le révélateur.

### 3.3. Par la méthode en tubes gel

- La CMI du révélateur en tubes gel peut être dérivée de celle trouvée en milieu liquide car la composition du milieu est identique à l'exception du gel. Les 2 concentrations seront choisies en fonction :
- de la facilité de lecture du virage à la plus faible concentration [C]1 de révélateur,
- du ralentissement de la croissance des souches à la plus forte concentration [C]2 de révélateur.

Tube gel 1 : une concentration de 20 mg/l permet la croissance de toutes les souches sans gêner la lecture.
Tube gel 2 : une concentration de 40 voire 50 mg/l de révélateur permet en l'absence d'inhibiteur spécifique de *M*. *tuberculosis* la croissance des souches de toutes les espèces à l'exception de *M*. *tuberculosis* qui est inhibé à cette concentration. Tube gel 3 : la concentration de révélateur associée à l'inhibiteur du tube est identique à celle du tube gel 1 (20 mg/l) ou du tube gel 2 (45 mg/l).

### EXEMPLE 2 : Définition d'un protocole simple de réalisation du test de dépistage et différenciation.

Pour échapper à l'effet inhibiteur du révélateur sur les souches du complexe *M*. *tuberculosis,* il est possible de séparer l'étape de croissance de l'étape de révélation. Pour cela, la croissance est assurée dans le milieu de culture ne contenant pas de révélateur. Puis, après un délai voisin de 14 jours d'incubation à 37° C nécessaire à l'obtention de la phase exponentielle de croissance, on ajoute extemporanément le révélateur ou mieux on met en contact le milieu de culture avec un gel concentré contenant le révélateur par agitation forte.
Le protocole de réalisation du test est cependant fastidieux car il nécessite une gestion des délais d'incubation et de lecture en fonction des tubes. Un protocole plus simple est proposé, en tenant compte de la CMI du révélateur.

### 1. Résultats en fonction de la concentration en révélateur

Des tubes Gel n° 1 contenant des concentrations croissantes (n = 9) de révélateur (de 22 à 57 mg/l) ont été ensemencés par 1 ml de milieu contenant 105 CFU/ml de chaque souche (n = 21) puis agités de suite (t0) après ensemencement.
La détermination de la concentration de révélateur à ajouter en tubes Gel n° 1 est présentée dans le tableau V ci-dessous.

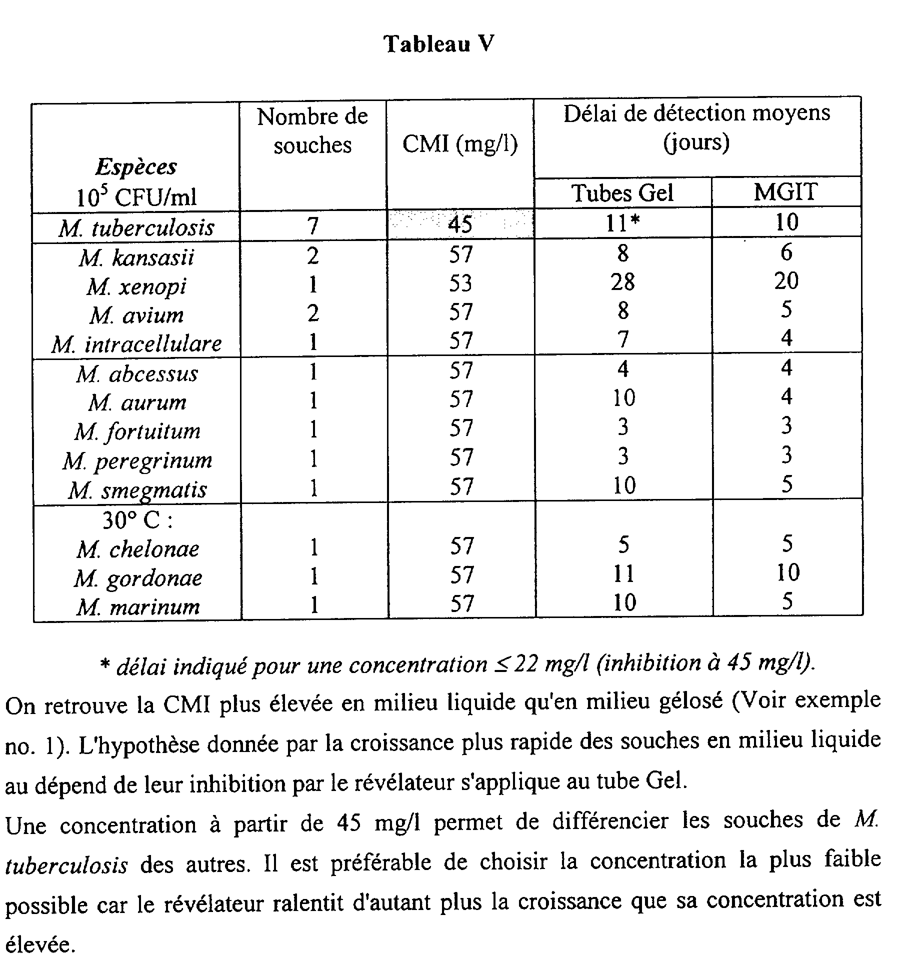

### 2. Résultats obtenus en utilisant des concentrations de révélateur en fonction de la CMI

15 souches ont été testées à 104 CFU/ml en tube 1 contenant 25 mg/l de révélateur et en tubes 2 (sans inhibiteur de *M. tuberculosis)* et 3 (avec inhibiteur des souches atypiques pathogènes) contenant 50 mg/l de révélateur.
Les résultats obtenus avec des concentrations de 25 et 50 mg/l sont résumés dans le Tableau VI ci-dessous.

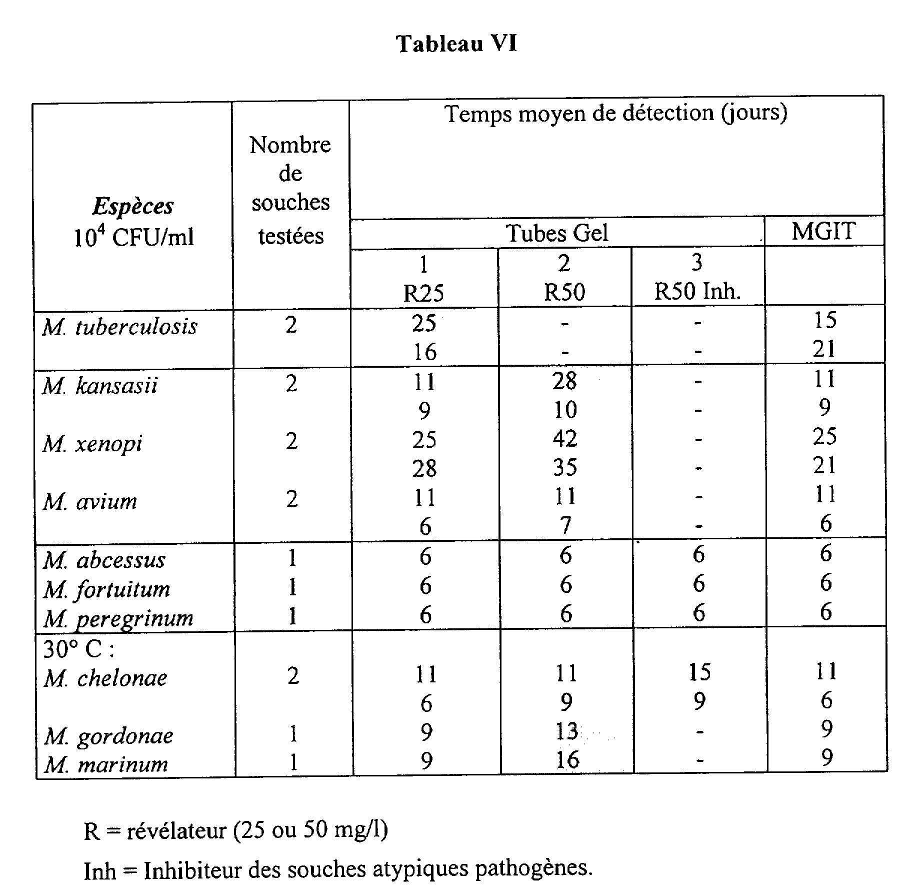

La concentration à 25 mg/l retarde la croissance de *M. tuberculosis.* Nous avons refait le test à 20 mg/l.
La concentration à 50 mg/1 retarde beaucoup la croissance des souches atypiques pathogènes et des souches poussant à 30° C. Nous avons donc refait l'essai en utilisant 40 mg/l.

Etude avec des concentrations de 20 et 40 mg/l
18 souches ont été testées à 105 CFU/ml en tube 1, 2 sans inhibiteur de *M*. *tuberculosis* et 3 avec inhibiteur des espèces atypiques pathogènes contenant respectivement 20, 40 et 20 mg/l. Les résultats sont présentés dans le Tableau VII ci-après.

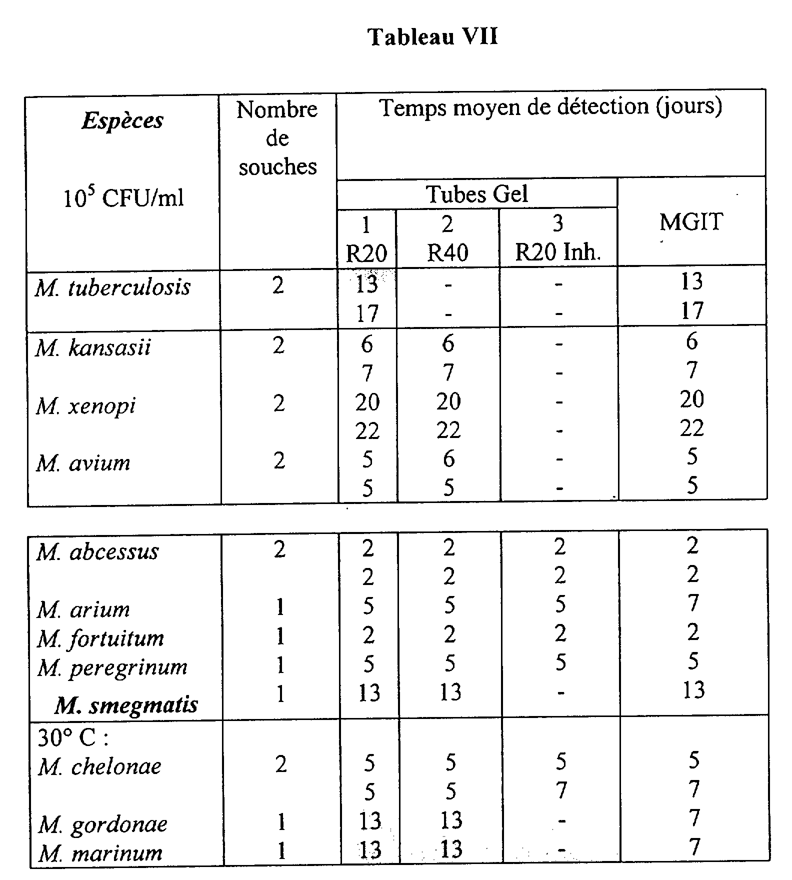

La croissance des souches est similaire à celle du tube MGIT excepté pour les souches poussant à 30° C. Cependant, avec des inoculi plus faibles, le temps de réponse augmente pour les souches pathogènes (dans le tube 1 pour *M*. *tuberculosis* et dans le tube 2 pour les autres).
Nous avons donc choisi d'ajouter du sérum de poulain à raison de 10 %.

Etude avec du sérum de poulain :
Plus l'inoculum diminue (de 106 à 103 CFU/ml), plus le sérum diminue le temps de détection quelle que soit la souche pathogène. Il en est de même pour les souches poussant à 30° C. Les résultats avec sérum sont donc similaires à ceux du tube MGIT quel que soit l'inoculum.

### 3. Conclusion

Le protocole de réalisation du test qui consiste à agiter les tubes après différents temps d'incubation se révèle fastidieux.

La méthode selon l'invention, plus simple, consiste à utiliser des tubes Gel agités de suite après l'addition de révélateur, et dont la concentration de révélateur est déduite de la CMI et permet une bonne croissance des souches que l'on veut identifier. La concentration de révélateur doit de préférence être :
- 22 mg/l dans le tube Gel 1 : une concentration supérieure inhibe la croissance de *M*. *tuberculosis* surtout.
- environ 45 mg/l dans le tube Gel 2 : une concentration inférieure en absence d'inhibiteur ne permet pas la différenciation des espèces et une concentration supérieure retarde la croissance de certaines souches pathogènes.
- 22 à 45 mg/l dans le tube Gel 3 : elle dépend de la concentration d'inhibiteur et doit donc être choisie en fonction de la CMI de l'inhibiteur.

### EXEMPLE 3 : Détermination de la CMI de l'inhibiteur utilisé dans le tube no. 3

### 1. Objectif

a) Déterminer pour chaque souche de mycobactérie, la CMI de l'inhibiteur utilisé dans le tube Gel 3 du test de dépistage-différenciation par la méthode des proportions.
   L'inhibiteur spécifique présent dans le tube Gel 3, le nitrite de sodium (NaNO2) inhibe la croissance des mycobactéries impliquées en pathologie respiratoire (*M*. *tuberculosis, M*. *xenopi, M*. *kansasii, M*. *avium, M. intracellulare*) à la différence des mycobactéries non pathogènes qui sont insensibles à son action.
   Il est donc nécessaire de déterminer la plus faible concentration de l'inhibiteur inhibant la croissance des mycobactéries pathogènes, en utilisant la méthode des proportions considérée comme méthode de référence.
b) Déterminer en parallèle, pour chaque souche de mycobactérie, la CMI de l'inhibiteur dans les conditions d'utilisation du tube Gel 3.
c) Déterminer la concentration de l'inhibiteur à ajouter dans le tube Gel 3 du test pour permettre la meilleure différenciation des espèces.

### 2. Matériels et méthodes

**Tableau VIII :**

| **liste des souches de l'étude (souches testées (n = 40)** | |
|---|---|
| **Espèce** | **Nombre de souches testées** |
| *M. tuberculosis* | 11 |
| | |
| **Mycobactéries atypiques pathogènes :** | |
| *M*. *avium* | 8 |
| *M. intracellulare* | 1 |
| *M*. *kansasii* | 9 |
| *M*. *xenopi* | 6 |
| | |

| **Mycobactéries atypiques non pathogènes :** | |
|---|---|
| *M*. *abscessus* | 1 |
| *M. chelonae* | 1 |
| *M. fortuitum* | 1 |
| *M. gordonae* | 1 |
| *M*. *peregrinum* | 1 |

### 2.1. Détermination de la CMI de l'inhibiteur par la méthode des proportions

La CMI de l'inhibiteur a été déterminée vis-à-vis de 23 des souches de l'étude par la méthode des proportions (méthode de référence) en milieu 7H11 contenant 10 % d'OADC.

La concentration minimale inhibitrice (CMI) de l'inhibiteur vis-à-vis d'une souche est définie comme la plus faible concentration pour laquelle le nombre de colonies sur la gélose est inférieur à 11 % par rapport au nombre de colonies présentes sur la gélose témoin sans inhibiteur.

La culture en milieu liquide, les inoculi, les milieux contenant le révélateur à différentes concentrations et la gamme de concentration du révélateur ont été décrits précédemment à l'exemple I, partie 2A.

Préparation des géloses contenant les différentes concentrations de l'inhibiteur:
Pour chaque souche testée, 400 ml de gélose sont préparés ; 8.4 g de gélose 7H11 sont mélangés à 360 ml d'eau distillée et 2 ml de glycérol. Le mélange est alors porté à ébullition de façon à obtenir une solution translucide.
Après stérilisation (autoclave 15 min à 121°C), la solution est ramenée à une température de 50 - 55°C ; 40 ml d'OADC sont alors additionnés et le tout est maintenu en surfusion à une température de 50 - 55°C.
200 µl de chaque concentration de l'inhibiteur sont mélangés de façon homogène à 20 ml du milieu gélosé maintenu en surfusion ; on obtient donc des concentrations finales de l'inhibiteur de 0.25 à 2 g/l. Le mélange est alors réparti en boîte de pétri à 4 compartiments, à raison de 5 ml par compartiment.
Une gélose ne contenant pas d'inhibiteur est également préparée (témoin de croissance).

Inoculation des géloses contenant l'inhibiteur:
Sur chaque gélose ainsi préparée, l'inoculum 10-3 est déposé sur 2 quadrants à raison de 50 µl par quadrant, et l'inoculum 10-5 est déposé sur 2 quadrants à raison de 50 µl par quadrant. Les cultures sont alors étalées à l'aide d'un râteau. Les géloses ainsi inoculées sont laissées sous la hotte pendant environ 10 minutes afin de les laisser sécher.

Les boîtes sont alors mises dans un sac à autoclave et placées à l'étuve à :
* 37°C pendant 3 et 7 jours pour :
   - *M. abscessus*
   - *M*. *fortuitum*
   - *M. peregrinum*
   - *M*. *smegmatis*
* 37°C pendant 15 et 21 jours pour :
   - *M*. *avium*
   - *M*. *intracellulare*
   - *M*. *kansasii*
* 37°C pendant 21 et 42 jours pour *M*. *tuberculosis*
* 37°C pendant 28 et 42 jours pour *M. xenopi*
* 30°C pendant 3 et 7 jours pour *M. chelonae*
* 30°C pendant 7 et 15 jour pour *M*. *gordonae*

Si la culture est insuffisante au bout du 1er temps d'incubation, garder les boîtes jusqu'au 2ème temps d'incubation.
On compte le nombre de colonies pour chacun des 2 inoculi sur la gélose témoin de croissance (sans inhibiteur). On compte le nombre de colonies pour chacun des 2 inoculi sur les géloses contenant les différentes concentrations en inhibiteur.

### 2.2. Détermination de la CMI de l'inhibiteur en tube gel

La CMI de l'inhibiteur vis-à-vis de 38 des souches de l'étude, a été déterminée en milieu du tube gel n° 3 contenant 45 mg/l de révélateur. Le tube a été ensemencé avec 0.5 ml d'une suspension à 0.5 McF de chacune des mycobactéries, diluée au 1:100 en tampon phosphate.
La croissance bactérienne est visualisée par le changement de couleur du milieu du violet au rose dans le capillaire. La CMI de l'inhibiteur vis-à-vis d'une souche est définie comme la plus faible concentration inhibant la croissance bactérienne.
Préparation des inoculi :
A partir d'une culture de mycobactéries sur milieu Lowenstein-Jensen, quelques colonies sont prélevées à l'aide d'une öese stérile.
Les colonies sont mises dans un tube falcon contenant des billes de verre. Après agitation manuelle pendant 1 à 2 minutes, 3 ml de sérum physiologique sont ajoutés. La suspension bactérienne ainsi récupérée est ajustée à 0.5 Mac Farland à l'aide de sérum physiologique stérile. Une dilution au 1/100 de cette suspension est réalisée en tampon phosphate (0.067M, pH 6.8) pour obtenir environ 106 bact/ml.

Préparation des tubes gel contenant l'inhibiteur à différentes concentrations :
Chaque tube gel contient 0.5 ml de milieu concentré contenant 90 mg/l de révélateur et l'inhibiteur à une concentration deux fois supérieure à la concentration finale désirée.

Les différentes concentrations finales d'inhibiteur testées sont les suivantes :
0.025 g/l - 0.050 g/l - 0.075 g/l - 0.1 g/l - 0.15 g/l - 0.2 g/l - 0.35 g/1 et 0.50 g/l.

Inoculation des tubes gel contenant l'inhibiteur :
Dans chaque tube gel sont introduits successivement : 20 µl du mélange des 7 antibiotiques, 50 µl de sérum de poulain puis 500 µl de l'inoculum préparé précédemment.

Les tubes gels sont observés tous les jours pendant 3 semaines puis 1 fois par semaine pendant 5 semaines, et la couleur du milieu au niveau du capillaire est notée.

La concentration minimale inhibitrice ou CMI de l'inhibiteur vis-à-vis d'une souche est déterminée comme la plus faible concentration de l'inhibiteur inhibant la croissance bactérienne : la croissance bactérienne est visualisée par le virage du violet au rose du révélateur au niveau du capillaire.

### 3. Résultats

### 3.1. Résultats obtenus avec la méthode des proportions décrite au point 2.1 ci-dessus

La CMI de l'inhibiteur vis-à-vis des 23 souches testées, déterminée par la méthode des proportions est donnée dans le tableau IX ci-après.

**Tableau IX :**

| **résultats de CMI de l'inhibiteur vis-à-vis des 23 souches testées par la méthode des proportions** | | |
|---|---|---|
| **Espèce** | **Souche** | **CMI (g/l)** |
| *M. tuberculosis* | H37Rv | 0,30 |
| | 4 | ≤ 0,25 |
| | 5 | 0,30 |
| | 7 | ≤ 0,25 |
| | 8 | 0,30 |
| | COET | ≤ 0,25 |

| **Mycobactéries atypiques pathogènes :** | | |
|---|---|---|
| *M*. *avium* | CIP | 0,80 |
| | BRIG | 1,0 |
| | ABO | 2,0 |
| | COD | 0,75 |
| | | |
| *M*. *intracellulare* | CIP | 2,0 |
| | | |
| *M. kansasii* | ATCC | 0,70 |
| | HUB | 0,75 |
| | VAL | 0,475 |
| | | |
| *M*. *xenopi* | BERN | 0,75 |
| | ATA | 0,70 |
| | CHI | 0,80 |
| | INC | 2,0 |
| | BLA | 2,0 |

| **Mycobactéries atypiques non pathogènes :** | | |
|---|---|---|
| *M*. *abscessus* | CIP | 2,0 |
| | | |
| *M. fortuitum* | CIP | > 2,0 |
| | | |
| *M. peregrinum* | CIP | > 2,0 |
| | | |
| *M*. *gordonae* | ANDR | 2,0 |

### 3.2. Résultats obtenus avec la méthode en tube gel décrite au point 2.2. ci-dessus

La CMI de l'inhibiteur vis-à-vis des 38 souches testées, déterminée par la méthode en tube gel n° 3 est donnée dans le tableau X ci-dessous.

**Tableau X**

| **Espèce** | **Souche** | **CMI (g/l)** |
|---|---|---|
| *M. tuberculosis* | LETI | ≤ 0,025 |
| | BERO | ≤ 0,025 |
| | COET | 0,075 |
| | 1 | ≤ 0,025 |
| | 2 | ≤ 0,025 |
| | 3 | < 0,025 |
| | 4 | ≤ 0,025 |
| | 5 | 0,075 |
| | 7 | ≤ 0,025 |
| | 8 | 0,05 |
| | 9 | 0,075 |

| **Mycobactéries atypiques pathogènes :** | | |
|---|---|---|
| *M. avium* | BEN | 0,15 |
| | ABO | 0,15 |
| | BUR | 0,35 |
| | CIP | 0,35 < CMI < 0,5 |
| | BRIG | 0,15 |
| | ZEM | 0,075 |
| | SANF | 0,15 |
| | POIN | 0,2 |
| | | |
| *M*. *kansasii* | HUB | 0,075 |
| | MAR | 0,2 |
| | MIS | 0,2 |
| | ATCC | 0,2 |
| | MEY | 0,15 |
| | SIL | 0,1 |
| | VIG | 0,15 |
| | VAL | 0,05 |
| | POU | 0,1 |
| | | |
| *M*. *xenopi* | CHI | 0,025 |
| | BLA | 0,05 |
| | ATA | 0,05 |
| | SAC | ≤ 0,025 |
| | BERN | 0,025 |
| | INC | 0,05 |

| **Mycobactéries atypiques non pathogènes :** | | |
|---|---|---|
| *M*. *abscessus* | CIP | > 0,5 |
| | | |
| *M*. *chelonae* | LOE | > 0,5 |
| | | |
| *M*. *fortuitum* | CIP | > 0,5 |
| | | |
| *M. peregrinum* | CIP | > 0,5 |

### 4. Conclusion

### 4.1. Par la méthode des proportions

- Toutes les souches de *M*. *tuberculosis* sont inhibées par l'inhibiteur à une concentration > 0.30 g/l (CMI ( 0.30 g/l).
- Les souches de mycobactéries atypiques responsables d'infections respiratoires sont inhibées par l'inhibiteur à une concentration variant de 0.475 à 2.0 g/l.
- Toutes les souches de mycobactéries atypiques non pathogènes sont insensibles à l'inhibiteur à partir d'une concentration de 2.0 g/l.
Comme dans le cas du révélateur (Voir exemple 1), nous avons remarqué que la taille des colonies était inversement proportionnelle à la concentration de l'inhibiteur.

### 4.2. Par la méthode en tube gel

- Dans l'exemple 1 précédent, nous avions remarqué que la croissance de certaines souches était ralentie à la plus forte concentration (45 mg/l) de révélateur. Il nous a donc semblé important de déterminer la CMI de l'inhibiteur en présence du révélateur utilisé à cette concentration dans le tube gel 3.
- Nous avions aussi remarqué que les CMI étaient plus élevées en tube gel qu'en milieu gélosé sans doute parce que la croissance des souches est plus rapide au dépend de l'inhibition. De façon surprenante, la CMI de l'inhibiteur en présence du révélateur du tube gel, est beaucoup plus faible qu'en milieu gélosé quelle que soit l'espèce et quelle que soit la souche de mycobactéries. Il semble que l'inhibition de la croissance bactérienne est augmentée par l'association agoniste inhibiteur-révélateur. Le révélateur potentialiserait l'effet de l'inhibiteur sur la croissance bactérienne. Il en résulte que l'étude en milieu gélosé aurait dû être réalisée en présence de révélateur pour que la différenciation des espèces soit interprétable.
- Toutes les souches du complexe *M*. *tuberculosis* sont inhibées par une concentration d'inhibiteur inférieure à 0.1 g/l dans le tube gel 3 en présence de révélateur à 45 mg/l (CMI ( 0.075 g/l).
- Toutes les souches de mycobactéries atypiques pathogènes sont inhibées par une concentration d'inhibiteur inférieure à 0.5 g/l dans le tube gel 3 (CMI < 0.5 g/l).
- Toutes les souches de mycobactéries atypiques non pathogènes sont insensibles à l'inhibiteur jusqu'à une concentration de 0.5 g/1 dans le tube 3 (CMI > 0.5 g/l).

En conclusion, une concentration d'inhibiteur de 0.5 g/l est sélectionnée dans le tube 3 pour inhiber la croissance des mycobactéries responsables de pathologies respiratoires (dont *M*. *tuberculosis)* sans inhiber les espèces non pathogènes.

### EXEMPLE 4 : Détermination de la CMI d'un inhibiteur spécifique de M. tuberculosis dans les conditions du tube-gel.

### 1. Objectif

a) Déterminer pour chaque souche de mycobactérie, la CMI d'un inhibiteur spécifique de *M*. *tuberculosis,* en présence de révélateur utilisé à une concentration permettant la croissance de toutes les souches de mycobactéries, dans les conditions d'utilisation du tube Gel 2.
b) Déterminer la plus faible concentration de l'inhibiteur à ajouter dans le tube Gel 2 pour inhiber la croissance du complexe *M*. *tuberculosis* sans retarder la croissance des autres souches pathogènes respiratoires.

### 2. Matériels et méthodes

**Tableau XI :**

| **liste des souches testées** | |
|---|---|
| **Espèce** | **Nombre de souches testées** |
| *M*. *tuberculosis* | 12 |
| | |
| **Mycobactéries atypiques pathogènes :** | |
| *M*. *avium* | 9 |
| *M*. *kansasii* | 9 |
| *M*. *xenopi* | 6 |
| | |
| **Mycobactéries atypiques non pathogènes :** | |
| *M*. *abscessus* | 1 |
| *M. fortuitum* | 1 |
| *M*. *peregrinum* | 1 |
| | |
| **Mycobactéries atypiques poussant à 30°C :** | |
| *M. chelonae* | 1 |
| *M*. *gordonae* | 1 |
| *M*. *marinum* | 1 |

Détermination de la CMI des inhibiteurs en tube gel :
La CMI des inhibiteurs vis-à-vis des souches de l'étude, a été déterminée en milieu gel contenant 22 mg/l de révélateur au lieu de 45 mg/l. Cette concentration correspond à celle utilisée dans le tube Gel 1 et choisie en fonction de la CMI du révélateur *pour M*. *tuberculosis* (voir exemple no. 2). Le tube a été ensemencé avec 0.5 ml d'une suspension à 0.5 McF de chacune des mycobactéries, diluée au 1:100 en tampon phosphate.
La croissance bactérienne est visualisée par le changement de couleur du milieu du violet au rose dans le capillaire. La CMI de l'inhibiteur vis-à-vis d'une souche est définie comme la plus faible concentration inhibant la croissance bactérienne.

Inhibiteur testé :
De nombreux inhibiteurs de *M*. *tuberculosis* sont décrits dans la littérature (27). Ces substances ont été utilisées pour l'identification des colonies isolées sur gélose. Seul le p-Nitro-(-Acetylamino-(-hydroxypropiophenone (NAP) a été utilisé sur le BACTEC( de Becton Dickinson pour différencier *M*. *tuberculosis* des autres espèces (32, 33).
Cependant, certaines souches dont *M*. *kansasii* ont montré des variations de sensibilité à cet inhibiteur (15).
Nous avons déterminé la CM1 du salicylate de sodium après sa sélection dans une première étude.

### 3. Résultats

La CMI du salicylate de sodium vis-à-vis des 42 souches de l'étude, déterminée par la méthode en tube Gel, est donnée dans le tableau XII ci-dessus.

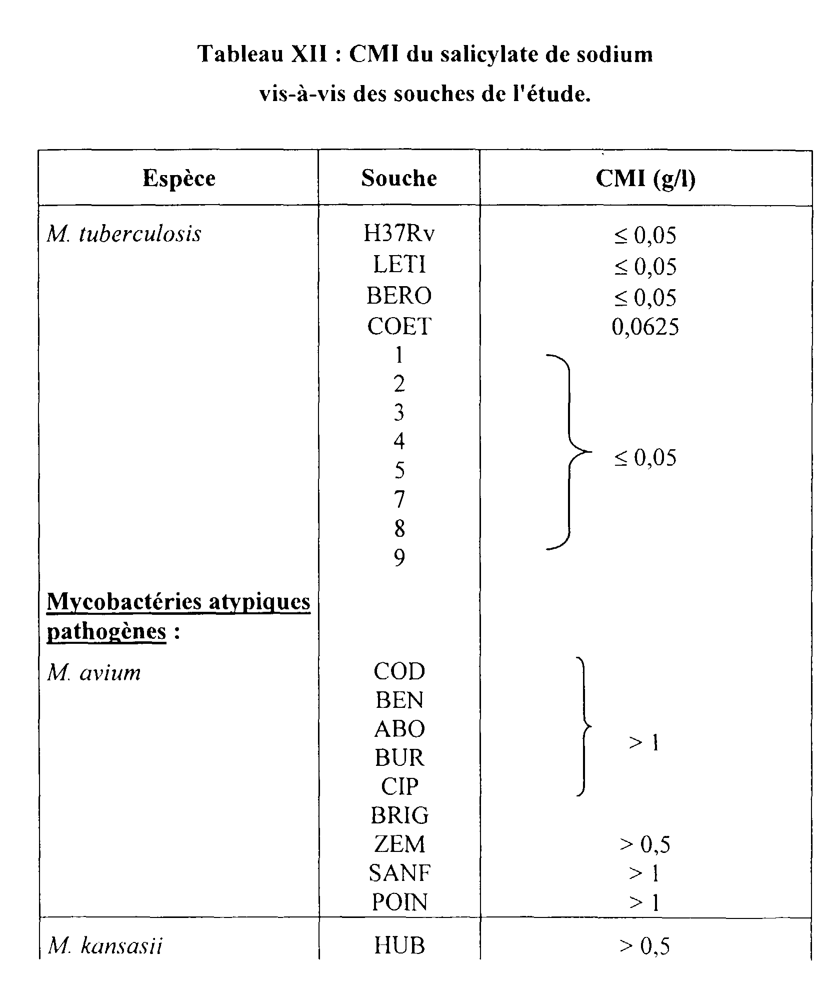

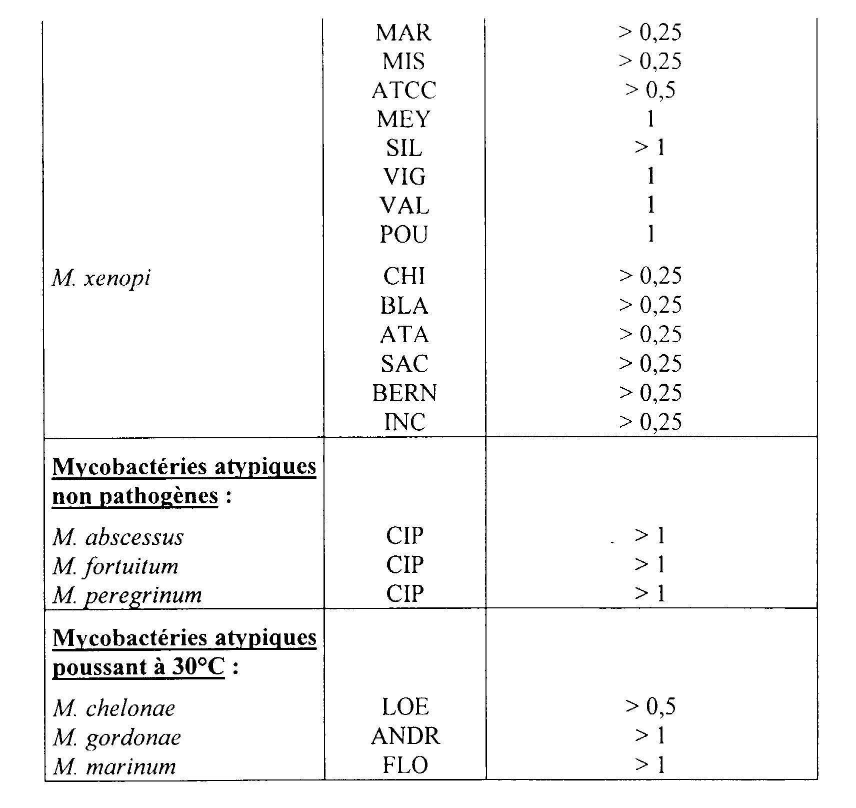

Choix du tube 2 : intérêt de l'association révélateur + inhibiteur :
Dans l'exemple précédent, nous avions remarqué que la croissance de certaines souches était ralentie à la plus forte concentration de révélateur (45 mg/l). L'écart entre la positivité du tube 1 et celle du tube 2 était voisin de 1 semaine.
Nous avons étudié le délai de détection en tube 1 et 2 lorsque l'inhibiteur sélectionné est associé à la plus faible concentration de révélateur (22 mg/l) (tableau XIII).

**Tableau XIII :**

| **délai de détection des souches en tubes 1 et 2** | | | | | |
|---|---|---|---|---|---|
| **Espèce** | **Nombre de souches** | **Inoculum UFC/ml** | **Délai de détection (jours)** | | |
| | | | Tube 1* | Tube 2* | MGIT |
| *M. tuberculosis* | 12 | 10⁶ | 9,0 | > 52 | 11,5 |
| | | 10⁴ | 15,5 | > 52 | 15,5 |
| *M*. *kansasii* | 9 | 10⁶ | 7,5 | 8,0 | 6,5 |
| | | 10⁴ | 13,5 | 15 | 10,5 |
| | | 10⁶ | 4,5 | 4,5 | 5,0 |
| *M*. *avium* | 9 | 10⁴ | 11,0 | 11,0 | 8,0 |
| | | 10⁶ | 12,5 | 12,5 | 13,5 |
| *M*. *xenopi* | 6 | 10⁶ | 12,5 | 12,5 | 13,5 |
| | | 10⁴ | 42,0 | 42,0 | > 42 |
| *M*. *abscessus* | | | | | |
| *M*. *fortuitum* | | 10⁶ | 5 | 5,5 | 4,5 |
| *M*. *peregrinum* | 3 | 10⁴ | 8 | 8 | 6 |
| *M*. *gordonae* | | | | | |
| *M*. *marinum* | | | | | |
| Tube 1 : révélateur : 22 mg/l | | | | | |
| Tube 2 : révévalteur : 22 mg/l + inhibiteur : 0.0625 g/l. | | | | | |

### 4. Conclusion

- Le salicylate de sodium est utilisable en présence du révélateur à 22 mg/l dans les conditions du tube Gel 2.
- Toutes les souches de *M. tuberculosis* sont inhibées par le salicylate de sodium à une concentration > 0.0625 g/l (CMI ( 0.0625 g/l). Les souches de mycobactéries atypiques non pathogènes, de *M*. *avium* et celles poussant à 30°C sont inhibées par l'inhibiteur à une concentration variant de 0.5 à 1.0 g/l.
   Les souches de *M*. *kansasii* et de *M*. *xenopi* sont inhibées par l'inhibiteur à une concentration variant de 0.25 à 1.0 g/l.
   En conclusion, une concentration d'inhibiteur de 0.0625 g/l est sélectionnée dans le tube 2 contenant 22 mg/l de révélateur, pour différencier les souches du *complexe M*. *tuberculosis* des mycobactéries atypiques pathogènes respiratoires.
- Le délai de détection des souches autres que celles du complexe *M. tuberculosis* est similaire en tube 1 et en tube 2. Le retard de positivité induit par l'inhibiteur n'excède pas 2 jours quelle que soit l'espèce et est toujours inférieur à une semaine quelle que soit la souche. L'espèce la plus sensible au salicylate semble être *M*. *kansasii.* Cependant, les 9 souches testées n'ont pas été inhibées à la concentration de 0.0625 g/l utilisée dans les conditions du tube 2.

### EXEMPLE 5 : Cinétique du temps de détection en fonction de la quantité de mycobactéries.

### 1. Objectif

Etude des résultats obtenus dans les 3 tubes gel 1, 2 et 3 avec :
- des souches cliniques et de collection à différents inoculi,
- des prélèvements surchargés à différents inoculi puis traités.

Le tube gel n° 1 de dépistage contient 22 mg/l de révélateur ; le tube gel n° 2 comporte l'association révélateur à même concentration que le tube 1 (22 mg/l) et inhibiteur spécifique du complexe *M. tuberculosis.* Le tube gel n° 3 comporte l'association de révélateur à 45 mg/l avec l'inhibiteur spécifique des souches atypiques pathogènes à 0,5 g/l (voir exemple 3 ci-dessus).
Etude de la stabilité et de la conservation des tubes Gel :
Le temps de détection peut être prolongé jusqu'à 6 semaines ou plus lorsque la concentration de mycobactéries de l'échantillon est faible. Nous avons donc étudié la stabilité des tubes gel dans ces conditions.

L'étude a porté sur : 1) 45 souches cliniques et de collection (10 espèces) à 106 et 104 UFC/ml, 2) des prélèvements cliniques respiratoires exempts de mycobactérie et surchargés par 103 à 106 UFC/ml de 15 souches (5 espèces) puis traités par la méthode à la N-acetylcystéine-soude. Des aliquotes de 0.5 ml ont été inoculés en tubes Löwenstein-Jensen (LJ), en tubes MGIT et en tubes gel. Des souches de bactéries contaminantes (12 espèces) ont été ensemencées dans les mêmes conditions pour vérifier l'efficacité du mélange antibiotique. Les tube ont tous été incubés pendant 8 semaines à 37° C (30° C dans certains cas) et observés chaque jour.

La croissance des mycobactéries est détectée plus rapidement par les méthodes en milieu liquide que par la méthode en milieu gélosé (voir exemples précédents). Les temps moyens de détection sont similaires en tube MGIT et en tube Gel 1 de dépistage en système purifié (tableau) ou de surcharge, quelle que soit la concentration bactérienne. Toutes les souches du complexe *M*. *tuberculosis* sont inhibées dans les tubes 2 et 3 alors que les autres espèces impliquées en pathologie respiratoire (*M*. *kansasii, M*. *avium* et *M. xenipi)* ne sont inhibées que dans le tube gel 3. Les mycobactéries atypiques non pathogènes sont détectées dans les 3 tubes. Les résultats des souches poussant à 30° C ne sont interprétables que dans le tube 1. La croissance des bactéries est mieux inhibée par le mélange antibiotique des tubes gel que par le PANTA du tube MGIT.

### 2- Matériels et méthodes

Souches testées (n = 45) :
Les souches de collection et cliniques de 10 espèces de mycobactéries (tableau XIV ci-après) ont été testées à 106 et 104 UFC/ml.

**Tableau XIV :**

| **liste des souches de l'étude** | | | |
|---|---|---|---|
| **Espèce** | **Nombre de souches testées** | | |
| | **Référence (CIP)** | | **Cliniques** |
| M. tuberculosis | 1 | H37 Rv = | 11 |
| | | CIP 64.31 = | |
| | | ATCC 27294 | |
| | | | |

| **Mycobactéries atypiques pathogènes :** | | | |
|---|---|---|---|
| *M. avium* | 1 | CIP 104244 = | 8 |
| | | ATCC 25291 = | |
| *M. kansasii* | 1 | CIP 104589 = | 8 |
| | | ATCC 12478 | |
| *M. xenopi* | - | | 6 |
| | | | |

| **Mycobactéries atypiques non pathogènes :** | | | |
|---|---|---|---|
| *M. abscessus* | 1 | CIP 104536 = | 1 |
| | | ATCC 19977 | |
| | | | |
| *M*. *chelonae* | - | | 1 |
| | | | |
| *M. fortuitum* | 1 | CIP 104 534 = | - |
| | | ATCC 6841 | |
| | | | |
| *M*. *gordonae* | - | | 2 |
| | | | |
| *M. marinum* | - | | 2 |
| | | | |
| *M. peregrinum* | 1 | CIP 105382 = | - |
| | | ATCC 14467 ou | |
| | | ATCC 23023 ou | |
| | | ATCC 23036 | |
| **TOTAL** | **6** | | **39** |

Prélèvements surchargés :
Des prélèvements tout-venant trouvés négatifs par la méthode conventionnelle ont été testés par la méthode MGIT (Becton Dickinson) après décontamination par Mycoprep pour éliminer les échantillons contaminés par des bactéries ou des champignons. 22 pools de prélèvements non contaminés ont été surchargés par 103 à 106 UFC/ml de 14 souches de mycobactéries (tableau XV ci-dessous) puis traités par la méthode N-acétyl-cystéine-soude.

**Tableau XV :**

| **liste des souches utilisées pour la surcharge des prélèvements cliniques.** | | | |
|---|---|---|---|
| **Espèces utilisées pour la surcharge des prélèvements cliniques** | **Nombre de souches testées** | | |
| | **Référence (CIP)** | | **Cliniques** |
| *M*. *tuberculosis* | 1 | H37 Rv | 2 |
| | | | |
| *M*. *kansasii* | 1 | CIP 104589 | 2 |
| | | | |
| *M*. *avium* | 1 | CIP 104244 | 2 |
| | | | |
| *M*. *xenopi* | - | | 4 |
| | | | |
| *M. abscessus* | - | | 1 |

### Méthodes :

### MGIT:

0.1 ml de PANTA lyophilisé repris en eau stérile et 0.5 ml d'OADC sont ajoutés dans chaque tube MGIT. Les tubes sont ensuite ensemencés avec 0.5 ml d'échantillon et incubés à 37°C (30°C dans certains cas). La lecture d'une fluorescence orange au fond du tube et au niveau du ménisque est effectuée à l'aide d'une lampe à U.V.

### SCREEN GEL mycobact :

Un mélange de sérum et d'antibiotiques (ou un comprimé dans certains essais) et 0.5 ml d'échantillon sont ajoutés dans chacun des 3 tubes Gel. Après incubation à 37°C (30°C dans certains cas), l'apparition d'une couleur rose franche est lue au niveau du gel, dans la partie capillaire du tube.

### 3. Résultats

### Test de dépistage :

### Souches de collection et cliniques :

Les temps moyens de détection sont calculés sur 3 à 4 déterminations différentes (par les repiquages, les inoculi, les lots de réactifs) effectuées par deux opérateurs. Les résultats sont similaires en tubes MGIT et en tube Gel 1 de dépistage lorsque l'inoculum est élevé (tableau XVI). Les temps de détection sont légèrement plus courts qu'en MGIT pour *M*. *tuberculosis* à 104 UFC/ml, par contre ils sont plus longs qu'en MGIT pour certaines souches de *M. avium* et de *M. xenopi* (tableau 3).

**Tableau XVI :**

| **temps de détection moyen obtenu avec des souches de collection et cliniques sur 3 déterminations en tube MGIT et en tube Gel de dépistage** | | | | |
|---|---|---|---|---|
| | **Temps moyen de détection (Jours)** | | | |
| **Espèces** | **10**^{**6**} **UFC/ml** | | **10**^{**4**} **UFC/ml** | |
| | **MGIT** | **Gel 1** | **MGIT** | **Gel 1** |
| *M*. *tuberculosis* (n = 12) | 9,5 ± 2,6 | 9,5 ± 2,6 | 16,5 ± 3,5 | 15,6 ± 3,0 |

| **Mycobactéries atypiques pathogènes respiratoires :** | | | | |
|---|---|---|---|---|
| | | | | |
| *M. kansasii* (n = 9) | 6,2 ± 2,2 | 7,6 ± 3,0 | 9,8 ± 2,5 | 13,1 ± 2,9 |
| *M. avium* (n = 7) | 4,7 ± 1,1 | 5,0 ± 1,4 | 8,4 ± 3,7 | 9,8 ± 3,1 |
| (n = 2) | | | 9,2 ± 3,4 | 19,4 ± 5,0 |
| *M*. *xenopie* (n = 6) | 13,3 ± 3,1 | 12,6 ± 1,9 | 37,8 ± 16,8 | 44,2 + 7,0 |

| **Mycobactéries atypiques non pathogènes poussant à 37°C :** | | | | |
|---|---|---|---|---|
| | | | | |
| *M. abscessus* (n = 2) | 3,0 | 4,2 | 3,3 | 4,0 |
| *M. fortuitum* (n = 1) | 2,4 | 3,0 | 4,6 | 4,2 |
| *M. peregrinum* (n = 1) | 4,0 | 6,5 | 5,5 | 12,5 |

| **Mycobactéries atypiques poussant à 30°C:** | | | | |
|---|---|---|---|---|
| | | | | |
| *M*. *chelonae* (n = 1) | 5,0 | 4,0 | 6,0 | 4,0 |
| *M. gordonae* (n = 2) | 5,8 | 5,3 | 10,0 | 15,5 |
| *M*. *marinum* (n *=* 2) | 5,0 | 6,0 | 8,7 | 8,7 |

### Prélèvements surchargés :

Les temps de détection sont légèrement plus courts avec *M*. *tuberculosis* et plus longs avec *M*. *xenopi* qu'en tube MGIT (tableau XVII). Il semble que le traitement de décontamination entraîne une inactivation de 1.5 log de *M*. *tuberculosis* et de 1 log de *M*. *xenopi.* Les autres espèces ne semblent pas sensibles à la décontamination.

**Tableau XVII :**

| **temps de détection obtenu avec des prélèvements surchargés par des souches de mycobactéries en tube MGIT et en tube Gel de dépistage.** | | | | | |
|---|---|---|---|---|---|
| **Espèces** | **Tube** | **Temps moyen de détection (Jours)** | | | |
| | | **10**^{**6**} **UFC/ml** | **10**^{**5**} **UFC/ml** | **10**^{**4**} **UFC/ml** | **10**^{**3**} **UFC/ml** |
| *M*. *tuberculosis* (n *=* 3) | MGIT | 12,7 | 17,3 | 24,3 | * |
| | Gel 1 | 13,7 | 16,3 | 19,7 | * |
| | | | | | |
| *M. kansasii* (n = 3) | MGIT | 7,7 | 11,0 | 13,7 | * |
| | Gel 1 | 8,0 | 12,7 | 13,7 | * |
| | | | | | |
| *M*. *avium* (n *=* 3) | MGIT | 4,3 | 6,3 | 8,3 | 11,0 |
| | Gel 1 | 5,0 | 7,0 | 9,7 | 12,5 |
| | | | | | |
| *M. xenopi* (n = 4) | MGIT | 18,0 | 22,0 | * | * |
| | Gel 1 | 22,0 | 34,0 | * | * |
| | | | | | |
| *M. abscessus* (n *=* 1) | MGIT | 3 | 3 | 4 | 4 |
| | Gel 1 | 3 | 3 | 4 | 4 |

### Test de différenciation :

Toutes les souches de *M*. *tuberculosis* sont inhibées dans les tubes 2 et 3. Celles de mycobactéries atypiques pathogènes respiratoires (*M*. *kansasii, M. avium, M*. *xenopi)* sont inhibées dans les tubes 3 (tableau XVIII ci-après). Les souches de mycobactéries atypiques non pathogènes sont insensibles aux inhibiteurs. Par contre, les souches poussant à 30°C ne donnent des résultats interprétables que dans le tube 1.

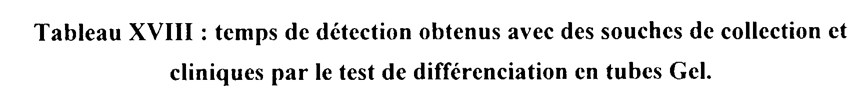

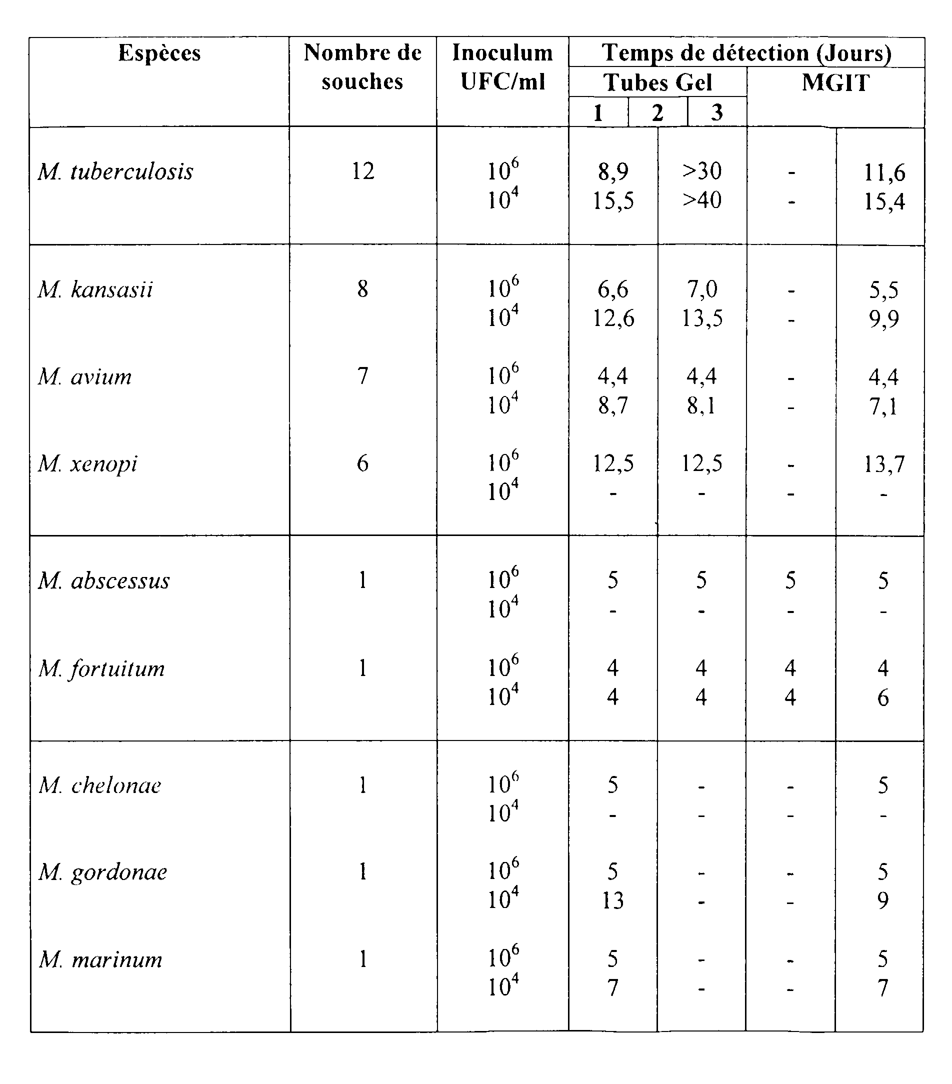

### 4. Conclusions

- Les résultats obtenus avec le tube Gel 1 de dépistage des mycobactéries sont très proches de ceux obtenus avec les tubes MGIT pour les souches isolées ou les prélèvements surchargés.
- L'association des 3 tubes gel permet la différenciation de *M. tuberculosis,* des espèces atypiques pathogènes respiratoires et des espèces non pathogènes.

Le test en tube gel permet donc :
1) le dépistage des mycobactéries aussi rapidement que la méthode MGIT et plus rapidement que la méthode conventionnelle,
2) la différenciation de *M*. *tuberculosis,* des mycobactéries atypiques impliquées en pathologie respiratoire et des espèces non pathogènes pour lesquelles seul un frottis de contrôle de la présence de BAAR est envisagé.
Le test en tubes gel limiterait le nombre d'examens complémentaires d'identification, il permettrait de ne s'attarder qu'aux espèces importantes en pathologie.

### REFERENCES

1. BIRD B.R., DENIISTON M.M., HUEBNER R.E. and GOOD R.C.1996. Changing practices in a mycobacteriology : a follow-up survey of state and territorial public health laboratories. J. Clin. Microbiol. 34 : 554-559.
2. BLOOM B.R. and MURRAY C.J.L. 1992. Tuberculosis : commentary on a reemergent killer. Science. 257 : 1055-1064.
3. BOUVET E. 1995. Epidémiologie de la tuberculose dans le monde. RFL. 273 : 53-56.
4. BRUNELLO F., FAVARI F. and FONTANA R. 1999. Comparison of the MB/Bac T and BACTEC 460 TB systems for recovery of mycobacteria from various clinical specimens. J. Clin. Microbiol. 37 : 1206-1209.
5. BURGUIERE A.M., POVEDA J.D. 1999. Diagnostic actuel de la tuberculose : méthodes traditionnelles ou biologie moléculaire. Option Bio. 222 (Suppl) 37-40.
6. CORNFIELD D.B., BEAVIS K.G., GREENE J.A., BOJAK M. and BONDI J. 1997. Mycobacterial growth and bacterial contamination in the Mycobacteria Growth Indicator Tube and BACTEC 460 culture systems. J. Clin. Microbiol. 35 : 2068-71.
7. DAVID H.L., LEVY-FREBAULT V., THOREL M.F. 1989. Méthodes de laboratoire pour mycobactériologie clinique. Institut Pasteur. Commission des Laboratoires d'Expertise et de Référence.
8. EL HELALI N. and VERGEZ P. 1993. Identification des mycobactéries. Feuillets de biologie XXXIV. 190 : 5-19.
9. EMILE C. 1998. Résistance du bacille tuberculeux aux antibiotiques : état des lieux. Option Bio. 214 : 15.
10. GOTO M., OKA S., OKUZUMI K., KIMURA S. and SHIMADA K. 1991. Evaluation of acridinium-ester-labeled DNA probes for identification of *Mycobacterium tuberculosis* and *Mycobacterium avium - Mycobacterium intracellulare* complex in culture. J. Clin. Microbiol. 29 : 2473-2476.
11. GROSSET J., TRUFFOT-PERNOT C., BOISVERT H. et LALANDE V. 1991. Qu'est ce que les mycobactéries atypiques ? Med. Mal. Infect. 21, Spécial : 7-15.
12. GROSSET J., TRUFFOT-PERNOT C., JARLIER V. 1993. Caractéristiques bactériologiques des mycobactéries du complexe aviaire. La Lettre de l'Infectiologue. Numéro hors série, 11-14.
13. HUANG Z.H., ROSS B.C., DWYER B. 1991. Identification of *Mycobacterium kansasii* by DNA hybridization. J. Clin. Microbiol. 29 : 2125-2129.
14. KUBICA G.P., KAUFMANN A.J., DYE W.E. 1964. Comments on the use of the new mucolytic agent, N-acetyl-L-cysteine-sodium hydroxide for culture of mycobacteria. Am. Rev. Respir. Dis. 89 : 284-286.
15. LEVY-FREBAULT V.V. 1992. Méthodes rapides de détection et de diagnostic des mycobactéries : Actualité et perspectives. Med. Mal. Infect. 22 : 391-406.
16. NOLTE F.S. and METCHOCK B. 1995. Mycobacterium, p. 400-437. In P.R. MURRAY, E.J. BARON, M.A. PFALLER, F.C. TENOVER and R.H. YOLKEN (ed.). Manuel of Clinical Microbiology, 6th Ed. ASM Press, Washington, D.C.
17. PALACI M., UEKI S.Y.M., SATO D.N., TELLES M.A.S., CURCIO M. and SILVA E.A.M. 1996. Evaluation of Mycobacteria Growth Indicator Tube for recovery and drug susceptibility testing of *Mycobacterium tuberculosis* isolates from respiratory specimens. J. Clin. Microbiol. 34 : 762-764.
18. PFYFFER G.E., WELSCHER H.M., and KISSLING P. 1997. Pretreatment of clinical specimens with sodium dodecyl (Lauryl) sulfate is not suitable for the Mycobacteria growth Indicator Tube cultivation method. J. Clin. Microbiol.
   35 : 2142-44.
19. RASKINE L., LELEU S., SANSON-LE PORS M.J. 1997. *Mycobacterium kansasii.* Aspects cliniques et bactériologiques. Feuillets de biologie.
   219 : 33-42.
20. RAVIGLIONE M. C., SNIDER D.E. and KOCHI A. 1995. Global epidemiology of tuberculosis. Morbidity and mortality of a worldwide epidemic. JAMA.
   273 : 220-226.
21. SALFINGER M., and MORRIS A.J. 1994. The role of the microbiology laboratory in diagnosing mycobacterial diseases. Am. J. Clin. Pathol.
   101 : (Suppl) S6-S13.
22. SNIDER D.E., RIVIGLIONE M. and KOCHI A. 1994. Global burden of tuberculosis, p. 3-11. In B.R. BLOOM (ed.), Tuberculosis : pathogenesis, protection and control. American Society for Microbiology, Washington D.C.
23. SOMOSKÔVI A., and MAGYAR P. 1999. Comparison of the Mycobacteria Growth Indicator Tube with MB Redox, Lôwenstein-Jensen, and Middlebrook 7H11 media for recovery of mycobacteria in clinical samples. J. Clin. Microbiol. 37 : 1366-1369.
24. STYRT B.A., SHINNICK T.M., RIDDERHOF J.C., CRAWFORD J.T. and TENOVER F.C. 1997. Turnaround times for mycobacterial cultures. J. Clin. Microbiol. 35 : 1041-1042.
25. TONNEL A.B. et les membres du Comité de Synthèse. 1995. La tuberculose en France : comment arrêter l'épidermie actuelle ? La lettre de l'Infectiologue, Tome X, 7 : 302-306.
26. TRUFFOT-PERNOT C. and GROSSET J. 1991. Diagnostic des infections à mycobactéries en 1991. Feuillets de Biologie. 182 : 27-33.
27. TSUKAMURA M. 1967. Identification of mycobacteria. Tubercle, Lond. 48, 311-338.
28. ALI-VEHMAS T., LOUHI M., and SANDHOLM M. 1991. Automation of the Resazurin Reduction test using fluorometry of microtitration trays. J. Vet. Med., 38, 358-372.
29. M. TSUKAMURA. Differentiation of Mycobacteria by susceptibility to hydroxylamine and 8-azaguanine. Jal of Bacteriology, 90, 556-557, 1965.
30. M. TSUKAMURA. Differentiation of *Mycobacterium tuberculosis* from other mycobacteria by sodum salicylate susceptibility. Amer. Rev. Resp. Dis., 86, 81-83, 1962.
31. M. TSUKAMURA. Differentiation of mycobacteria by susceptibility to nitrite and propylene glycol. Am. Review of Resp. Disease, 98, 505-506, 1968.
32. A. LASZLO and S. SIDDIQI. Evaluation of a rapid radiometric differentiation test for the *Mycobacterium tuberculosis* complex by selective inhibition with P-Nitro-(-Acetyl-amino-(-hydroxypropiophenone. Jal of Clin. Microbiol. 19, 694-698, 1984.
33. W.M. GROSS and J.E. HAWKINS. Radiometric selective inhibition tests for differentiation of *Mycobacterium tuberculosis, Mycobacterium bovis,* and other mycobacteria. Jal of Clin. Microbiol. 21, 565-568, 1985.

## Revendications

1. Méthode de différenciation du complexe *M. tuberculosis* des autres mycobactéries atypiques dans un échantillon biologique, comprenant les étapes consistant à :
a) ensemencer simultanément deux aliquotes d'un échantillon à tester dans
- un tube gel n° 1, comprenant dans une phase gel stérile :
(i) un milieu de culture pour mycobactéries additionné d'un mélange d'antibiotiques pour inhiber les bactéries contaminantes,
(ii) de la résazurine à une concentration C1 inférieure à la CMI pour le complexe *M*. *tuberculosis* et suffisante pour révéler la présence de microorganismes dans l'échantillon,
- un tube gel n° 2, comprenant dans une phase gel stérile :
(i) un milieu de culture pour mycobactéries additionné d'un mélange d'antibiotiques pour inhiber les bactéries contaminantes.
(ii) soit de la résazurine à une concentration C2 supérieure ou égale à la CMI pour le complexe *M. tuberculosis,* et inférieure à la CMI pour les autres espèces de mycobactéries, soit de la résazurine à une concentration C'2 inférieure ou égale à la CMI pour le complexe *M. tuberculosis* à une concentration suffisante pour révéler la présence de microorganismes dans l'échantillon, et un inhibiteur spécifique du complexe *M. tuberculosis* à une concentration suffisante pour inhiber sa croissance,
b) agiter immédiatement les tubes 1 et 2 après l'étape d'ensemencement pour mélanger l'échantillon et le gel,
c) incuber les tubes n° 1 et 2 à 37° C et procéder à la lecture des tubes.

2. Méthode selon la revendication 1 caractérisée en ce que l'on ajoute de préférence dans le tube gel n°2 de la résazurine à une concentration C'2 inférieure ou égale à la CMI pour le complexe *M*. *tuberculosis* à une concentration suffisante pour révéler la présence de microorganismes dans l'échantillon, et un inhibiteur spécifique du complexe *M*. *tuberculosis* à une concentration suffisante pour inhiber sa croissance.

3. Méthode de différenciation des mycobactéries atypiques impliquées en pathologie respiratoire des autres espèces non pathogènes dans un échantillon biologique comprenant les étapes consistant à :
a) ensemencer simultanément deux aliquotes d'un échantillon à tester dans
- un tube gel n° 1, comprenant dans une phase gel stérile :
(i) un milieu de culture pour mycobactéries additionné d'un mélange d'antibiotiques pour inhiber les bactéries contaminantes,
(ii) de la résazurine à un concentration C1 inférieure à la CMI pour le complexe *M. tuberculosis* et suffisante pour révéler la présence de microorganismes dans l'échantillon,
- un tube gel n° 3, comprenant dans une phase gel stérile :
(i) un milieu de culture pour mycobactéries additionné d'un mélange d'antibiotiques pour inhiber les bactéries contaminantes,
(ii) de la résazurine à l'intérieur du gel à une concentration C3 supérieure à C1 et un inhibiteur spécifique des mycobactéries à une concentration C4, C3 et C4 étant choisies de façon à inhiber les mycobactéries atypiques pathogènes éventuellement présentes dans l'échantillon, sans affecter la croissance des mycobactéries atypiques non pathogènes, et C3 étant une concentration habituellement utilisée pour révéler la présence de microorganismes dans un échantillon,
b) agiter immédiatement les tubes 1 et 3 après l'étape a) d'ensemencement pour mélanger l'échantillon et le gel,
c) incuber les tubes n° 1 et 3 à 37° C et procéder à la lecture des tubes.

4. Méthode de détection et de différenciation des mycobactéries du complexe *M*. *tuberculosis* des mycobactéries pathogènes et des autres espèces atypiques non pathogènes, ladite méthode consistant à :
a) ensemencer simultanément trois aliquotes d'un même échantillon dans des tubes gel 1, 2 et 3 selon les revendications 1 à 3,
b) agiter les tubes immédiatement après ensemencement et
c) incuber les tubes à 37° C et procéder à la lecture des tubes.

5. Méthode selon l'une quelconque des revendications précédentes permettant en outre de détecter des mycobactéries atypiques poussant à 30° C en cas d'infection cutanée, caractérisée en ce qu'on ensemence un aliquote de l'échantillon dans un tube 1 supplémentaire dans l'étape a) et on incube ledit tube supplémentaire à 30° C dans l'étape c).

6. Méthode selon la revendication 1, caractérisée en ce que la concentration C1 en résazurine est comprise entre 18 et 25 mg/l et la concentration C2 comprise entre 30 et 50 mg/l, préférentiellement comprise entre 40 et 50 mg/l.

7. Méthode selon la revendication 1, caractérisée en ce que la concentration C1 est de 22 mg/l.

8. Méthode selon la revendication 1, caractérisée en ce que la concentration C2 est de 45 mg/l.

9. Méthode selon l'une des revendications 1 et 2, caractérisée en ce que la concentration C'2 en résazurine est égale à C1.

10. Méthode selon la revendication 1, caractérisée en ce que l'inhibiteur spécifique du complexe *M. tuberculosis* est le salicylate de sodium.

11. Méthode selon la revendication 10, caractérisée en ce que la concentration C'2 en résazurine et la concentration en salicylate de sodium sont respectivement de 22 mg/l et 62,5 mg/l.

12. Méthode selon la revendication 3, caractérisée en ce que la concentration C3 en résazurine est comprise entre 40 et 50 mg/l.

13. Méthode selon la revendication 3, caractérisée en ce que C3 est de 45 mg/l.

14. Méthode selon la revendication 3, caractérisée en ce que l'inhibiteur des mycobactéries pathogènes est le nitrite de sodium (NaNO2).

15. Méthode selon la revendication 14, caractérisée en ce que la concentration C4 en NaNO2 est comprise entre 0,2 et 0,5 g/l.

16. Méthode selon la revendication 15, caractérisée en ce que les concentrations C3 et C4 du mélange résazurine/NaNO2 sont respectivement de 45 mg/l et 0,5 g/l.

17. Méthode selon la revendication 4, caractérisée en ce que les tubes 1, 2 et 3 contiennent les concentrations finales respectives suivantes :
- tube n° 1 : résazurine = 22 mg/l
- tube n° 2 : résazurine = 45 mg/l ou résazurine/salicylate de sodium : 22 mg/l/62,5 mg/l.
- tube n° 3 : résazurine/nitrite de sodium = 45 mg/1/0,5 g/l.

18. Méthode selon l'une quelconque des revendications 1 à 4, caractérisée en ce que les antibiotiques sont formulés sous forme de comprimés reconstitués dans la phase gel extemporanément.

19. Méthode selon l'une quelconque des revendications 1 à 4, caractérisée en ce que les tubes gel contiennent en outre du sérum de poulain.

20. Méthode selon la revendication 19, caractérisée en ce que la concentration en sérum est de 5 % par tube.

21. Méthode selon la revendication 20, caractérisée en ce que le sérum est compris dans le comprimé d'antibiotiques.

22. Kit pour la mise en oeuvre d'une méthode selon l'une quelconque des revendications 1 à 4, caractérisé en ce qu'il comprend :
- un tube gel n° 1, comprenant dans une phase gel stérile :
(i) un milieu de culture pour mycobactéries additionné d'un mélange d'antibiotiques pour inhiber les bactéries contaminantes.
(ii) de la résazurine à une concentration C1 inférieure à la CMI pour le complexe *M. tuberculosis* et suffisante pour révéler la présence de microorganismes dans l'échantillon, et
- un tube gel n° 2, comprenant dans une phase gel stérile :
(i) un milieu de culture pour mycobactéries additionné d'un mélange d'antibiotiques pour inhiber les bactéries contaminantes.
(ii) de la résazurine à une concentration C2 supérieure ou égale à la CMI pour le complexe *M. tuberculosis* et inférieure à la CMI pour les autres espèces de mycobactéries, ou
(ii)' de la résazurine à une concentration C'2 inférieure ou égale à la CMI pour le complexe *M. tuberculosis* et à une concentration suffisante pour révéler la présence de microorganismes dans l'échantillon, et un inhibiteur spécifique du complexe *M*. *tuberculosis* à une concentration suffisante pour inhiber sa croissance, et/ou
- un tube gel n° 3, comprenant dans une phase gel stérile :
(i) un milieu de culture pour mycobactéries additionné d'un mélange d'antibiotiques pour inhiber les bactéries contaminantes.
(ii) de la résazurine à l'intérieur du gel à une concentration C3 supérieure à C1 et un inhibiteur spécifique des mycobactéries à une concentration C4, C3 et C4 étant choisies de façon à inhiber les mycobactéries atypiques pathogènes éventuellement présentes dans l'échantillon, sans affecter la croissance des mycobactéries atypiques non pathogènes, et C3 étant une concentration habituellement utilisée pour révéler la présence de microorganismes dans un échantillon.

23. Kit pour la mise en oeuvre selon l'une quelconque des revendications 1 à 5, caractérisé en ce qu'il comprend un tube gel n° 1 supplémentaire pour la détection de mycobactéries poussant à 30° C.
